# EUROPEAN PATENT APPLICATION

(11) **EP 4 024 116 A1**
(43) Date of publication of application: **06.07.2022**
(21) Application number: 20856665.3
(22) Date of filing: 24.08.2020
(51) Int. Cl.: G02B 23/26, A61B 1/00, A61B 1/12

(54) **ENDOSCOPE**

(30) Priority: 27.08.2019 JP 2019154814; 28.05.2020 JP 2020093450
(71) Applicant: FUJIFILM Corporation, Tokyo 106-8620 (JP)
(72) Inventor: YOSHIOKA, Masato, Ashigarakami-gun, Kanagawa 258-8538 (JP); SUZUKI, Hiroaki, Ashigarakami-gun, Kanagawa 258-8538 (JP); NOKO, Motoki, Ashigarakami-gun, Kanagawa 258-8538 (JP); TATSUTA, Takeichi, Ashigarakami-gun, Kanagawa 258-8538 (JP)
(74) Representative: Hughes, Andrea Michelle
(86) International application number: PCT/JP2020/031846
(87) International publication number: WO 2021/039719

(57) **Abstract**

There is provided an endoscope that can efficiently remove the dirt of an auxiliary light-emitting window.

A distal end part (12d) of an insertion part of an endoscope is provided with a distal end surface (56), an objective lens (21) that is disposed on the distal end surface (56), an air/water supply nozzle (25) that jets fluid toward the objective lens (21), and a lens (23) for auxiliary measurement light that is disposed on the distal end surface (56) and emits auxiliary measurement light. The lens (23) for auxiliary measurement light is disposed between the objective lens (21) and the air/water supply nozzle (25) in a fluid jet range of the air/water supply nozzle (25).

## Description

### BACKGROUND OF THE INVENTION

### 1. Field of the Invention

The present invention relates to an endoscope that emits auxiliary measurement light.

### 2. Description of the Related Art

A distance to an object to be observed, the size of the object to be observed, and the like are acquired in the field of an endoscope. In, for example, WO2018/051680A, a subject is irradiated with auxiliary measurement light from an auxiliary light-emitting window provided at a distal end part of an endoscope and a spot is formed the subject. Accordingly, a processor device for an endoscope specifies the position of the spot from a picked-up image that is obtained from the image pickup of the subject. Then, the processor device for an endoscope detects an observation distance from the position of the spot.

On the other hand, the distal end part of the endoscope is provided with an observation window, an illumination window, a fluid jet nozzle, and the like in addition to the auxiliary light-emitting window. The fluid jet nozzle jets fluid, such as washing water, to wash the observation window. Since the illumination window is disposed in the jet range of the fluid jet nozzle in the endoscope disclosed in JP2009-39462A, washing water jetted from the fluid jet nozzle is blown to the surface of the illumination window. Accordingly, the heat of the illumination window, which is generated due to light irradiation, can be dissipated.

### SUMMARY OF THE INVENTION

Since the distal end part of the endoscope gets dirty due to body fluid and the like, it is desired to wash the auxiliary light-emitting window as well as the observation window. However, in JP2009-39462A, washing water is blown to the illumination window for heat dissipation, and the washing of the illumination window is not considered. Accordingly, even though the auxiliary light-emitting window is disposed instead of the illumination window in the same configuration as that disclosed in JP2009-39462A, it is not possible to deal with the washing of the auxiliary light-emitting window and the removal of dirt is insufficient.

The present invention has been made in consideration of the above-mentioned circumstances, and an object of the present invention is to provide an endoscope that can efficiently remove the dirt of an auxiliary light-emitting window.

An endoscope according to an aspect of the present invention comprises an insertion part, a distal end surface, an observation window, a fluid jet nozzle, and an auxiliary light-emitting window, and the auxiliary light-emitting window is disposed between the observation window and the fluid jet nozzle in a fluid jet range of the fluid jet nozzle. The insertion part is inserted into an object to be examined. The distal end surface is provided at a distal end of the insertion part. The observation window is disposed on the distal end surface. The fluid jet nozzle is disposed on the distal end surface and jets fluid toward the observation window. The auxiliary light-emitting window is disposed on the distal end surface and emits auxiliary measurement light.

It is preferable that the fluid jet nozzle jets liquid or gas as the fluid and, in a case where the liquid or the gas is jetted toward the observation window from the fluid jet nozzle, a flow velocity of the liquid at a position where the liquid reaches the observation window is 2 m/s or more and a flow velocity of the gas at a position where the gas reaches the observation window is 40 m/s or more.

It is preferable that an optical member for an auxiliary light-emitting window, which forms the auxiliary light-emitting window, includes a notched portion provided at a position facing the fluid jet nozzle.

It is preferable that the notched portion is disposed at a position avoiding an optical path of the auxiliary measurement light in a case where the auxiliary measurement light is emitted from the auxiliary light-emitting window.

It is preferable that the fluid jet nozzle is assembled in the insertion part in a state where the fluid jet nozzle is in contact with the notched portion.

It is preferable that the notched portion is in contact with the fluid jet nozzle, so that a position of the auxiliary light-emitting window in an axial direction of the insertion part is restricted.

It is preferable that the endoscope further comprises: a distal end part body that holds an optical member for an auxiliary light-emitting window forming the auxiliary light-emitting window, an image pickup optical system including the observation window, and the fluid jet nozzle; and a distal end cap that covers a distal end side of the distal end part body, and the optical member for an auxiliary light-emitting window includes a notched portion that is provided at a position facing the distal end cap.

It is preferable that the notched portion is in contact with the distal end cap, so that a position of the auxiliary light-emitting window in an axial direction of the insertion part is restricted.

It is preferable that the optical member for an auxiliary light-emitting window is formed in a columnar shape and the notched portion is an inclined surface which is inclined toward an outer peripheral surface from a distal end of the optical member for auxiliary measurement light.

It is preferable that an outer diameter of the auxiliary light-emitting window is 0.5 mm or more and 1.6 mm or less, a first minimum distance, which is a minimum distance between an outer peripheral edge of the observation window and an outer peripheral edge of the auxiliary light-emitting window, is 0 mm or more and 1.5 mm or less, and a second minimum distance, which is a minimum distance between the outer peripheral edge of the auxiliary light-emitting window and a distal end of the fluid jet nozzle, is 0 mm or more and 0.5 mm or less.

It is preferable that a mounting position for the fluid jet nozzle on the distal end surface and a distal end surface of the auxiliary light-emitting window are disposed at a same position in an axial direction of the insertion part, and a distal end surface of the observation window is positioned on a distal end side of the distal end surface of the auxiliary light-emitting window in the axial direction and includes a guide surface continuous between an outer peripheral edge of the auxiliary light-emitting window and an outer peripheral edge of the observation window.

It is preferable that an opening width of the fluid jet nozzle is smaller than an outer diameter of the observation window and the guide surface is positioned in the fluid jet range of the fluid jet nozzle.

It is preferable that an outer diameter of the auxiliary light-emitting window is smaller than an outer diameter of the observation window.

According to the present invention, it is possible to efficiently remove the dirt of an auxiliary light-emitting window.

### BRIEF DESCRIPTION OF THE DRAWINGS

Fig. 1 is a diagram showing the appearance of an endoscope system.
Fig. 2 is a plan view of a distal end part of an endoscope.
Fig. 3 is a block diagram showing the functions of the endoscope system.
Fig. 4 is a schematic diagram showing the configuration of an auxiliary measurement light-emitting unit.
Fig. 5 is a diagram illustrating a state where an insertion part of the endoscope is inserted into an object to be examined.
Fig. 6 is a diagram illustrating a relationship between the distal end part of the endoscope and a near end PN, an intermediate vicinity PM, and a far end PF in a range R1 of an observation distance.
Fig. 7 is a cross-sectional view of the distal end part of the endoscope.
Fig. 8 is a perspective view of the distal end part of the endoscope.
Fig. 9 is a cross-sectional view of the main portion of the distal end part of the endoscope.
Fig. 10 is a plan view of the distal end part of the endoscope, and is a plan view showing a dimensional relationship between an auxiliary light-emitting window, an observation window, and a fluid jet nozzle.
Fig. 11 is a plan view of the distal end part of the endoscope, and is a plan view showing a state where fluid jetted from the fluid jet nozzle is diffused on a guide surface.
Fig. 12 is a plan view of a distal end part of an endoscope that is not provided with a guide surface as a comparative example.
Fig. 13 is a cross-sectional view of a distal end part of an endoscope according to a second embodiment.
Fig. 14 is a perspective view showing a positional relationship between a fluid jet nozzle and an auxiliary light-emitting window of the second embodiment.
Fig. 15 is an exploded perspective view of the distal end part of the endoscope according to the second embodiment.
Fig. 16 is a perspective view showing a positional relationship between the fluid jet nozzle and the auxiliary light-emitting window in a modification example of the second embodiment.
Fig. 17 is a cross-sectional view of a distal end part of an endoscope according to a third embodiment.
Fig. 18 is a perspective view showing a positional relationship between a distal end cap and an auxiliary light-emitting window of the third embodiment.
Fig. 19 is an image diagram showing a spot and a first measurement marker in a case where an observation distance corresponds to the near end PN.
Fig. 20 is an image diagram showing a spot and a first measurement marker in a case where an observation distance corresponds to the intermediate vicinity PM.
Fig. 21 is an image diagram showing a spot and a first measurement marker in a case where an observation distance corresponds to the far end PF.
Fig. 22 is an image diagram of an example of a measurement image in which a measurement marker is superimposed on a subject image.
Fig. 23 is an image diagram of an example of a measurement image in which a measurement marker is superimposed on a subject image.
Fig. 24 is an image diagram of an example of a measurement image in which a measurement marker is superimposed on a subject image.
Figs. 25A, 25B, and 25C are diagrams showing types of measurement markers, Fig. 25A shows a measurement marker that includes a line segment and gradations on the left side of a spot SP, Fig. 25B shows a measurement marker that includes a line segment and gradations on the lower side of a spot SP, and Fig. 25C shows a measurement marker that includes a line segment and gradations on the upper right side of a spot SP.
Fig. 26 is a diagram illustrating first measurement markers having a cruciform shape, a cruciform shape with gradations, a distorted cruciform shape, a circular-and-cruciform shape, and the shape of a measurement point group.
Fig. 27 is an image diagram showing three concentric circular markers having the same color.
Fig. 28 is an image diagram showing three concentric circular markers having different colors.
Fig. 29 is an image diagram showing distorted concentric circular markers.
Fig. 30 is a diagram illustrating a light emission pattern in which spotlight is intermittently applied.
Fig. 31 is an image diagram showing an intersection line and gradations.
Fig. 32 is a diagram illustrating a light emission pattern in which line-like measurement light is intermittently applied.
Fig. 33 is a diagram illustrating stripe pattern light ZPL.
Fig. 34 is a diagram illustrating the light emission patterns of stripe pattern light ZPL having a phase X, stripe pattern light ZPL having a phase Y, and stripe pattern light ZPL having a phase Z.
Fig. 35 is a diagram illustrating grid-pattern measurement light LPL.
Fig. 36 is a diagram illustrating a light emission pattern in which the grid-pattern measurement light is intermittently applied.
Fig. 37 is a diagram illustrating three-dimensional plane light TPL.
Fig. 38 is a diagram illustrating a light emission pattern in which three-dimensional plane light is intermittently applied.

### DESCRIPTION OF THE PREFERRED EMBODIMENTS

### [First embodiment]

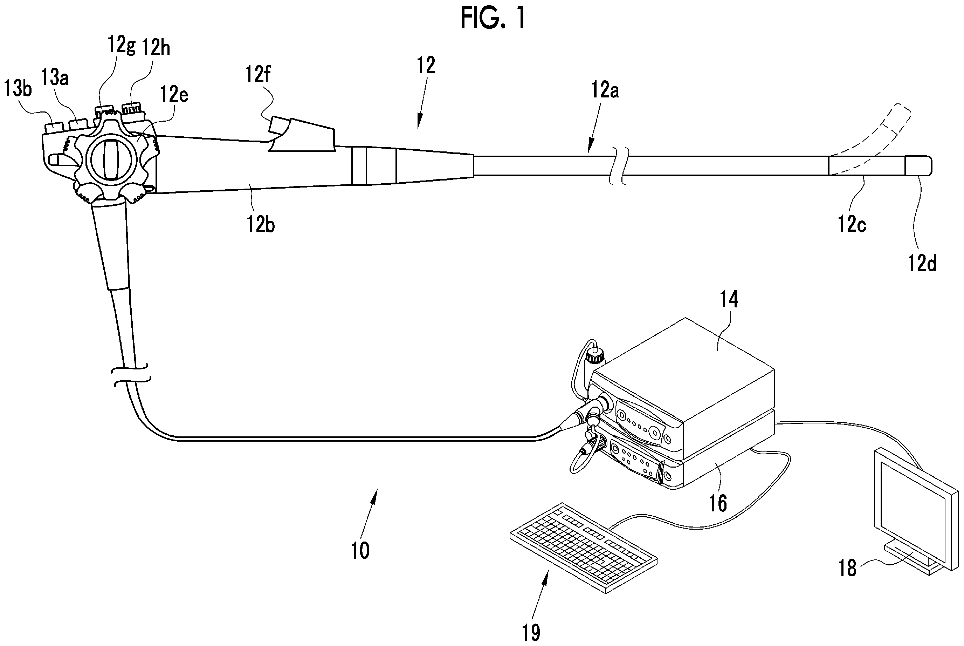

As shown in Fig. 1, an endoscope system 10 includes an endoscope 12, a light source device 14, a processor device 16, a monitor 18, and a user interface 19. The endoscope 12 is optically connected to the light source device 14, and is electrically connected to the processor device 16. The processor device 16 is electrically connected to the monitor 18 (display unit) that displays an image. The user interface 19 is connected to the processor device 16 and is used for various setting operations and the like for the processor device 16. The user interface 19 includes a mouse and the like in addition to a keyboard shown in Fig. 1.

The endoscope 12 includes an insertion part 12a that is to be inserted into an object to be examined, an operation part 12b that is provided at the proximal end portion of the insertion part 12a, and a bendable part 12c and a distal end part 12d that are provided on the distal end side of the insertion part 12a. The bendable part 12c operates to be bent by the operation of angle knobs 12e of the operation part 12b. The distal end part 12d is made to face in a desired direction by the bending operation of the bendable part 12c.

The endoscope 12 has a normal mode and a length measurement mode, and these two modes are switched by a mode changeover switch 13a that is provided on the operation part 12b of the endoscope 12. The normal mode is a mode where an object to be observed is illuminated with illumination light. In the length measurement mode, an object to be observed is illuminated with illumination light or auxiliary measurement light and a measurement marker to be used for the measurement of the size and the like of the object to be observed is displayed in a picked-up image obtained from the image pickup of the object to be observed. The auxiliary measurement light is light that is used to measure a subject.

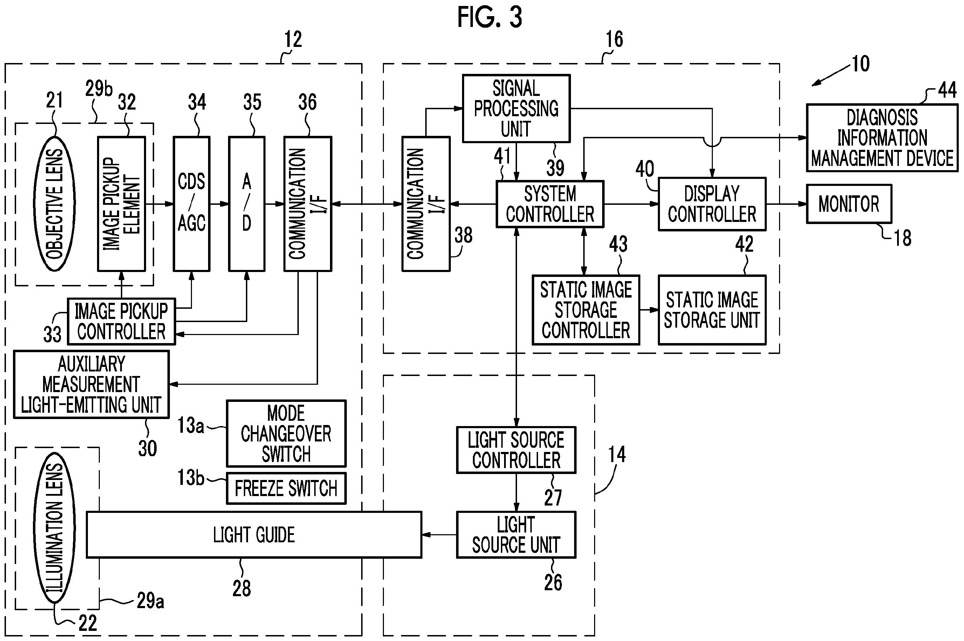

Further, the operation part 12b of the endoscope 12 is provided with a freeze switch 13b (static image-acquisition instruction unit) that is used to give a static image-acquisition instruction to acquire the static image of a picked-up image. In a case where a user operates the freeze switch 13b, the screen of the monitor 18 is frozen and displayed and an alert sound (for example, "beep") informing the acquisition of a static image is generated together. Then, the static images of the picked-up image, which are obtained before and after the operation timing of the freeze switch 13b, are stored in a static image storage unit 42 (see Fig. 3) provided in the processor device 16.

The static image storage unit 42 is a storage unit, such as a hard disk or a universal serial bus (USB) memory. In a case where the processor device 16 can be connected to a network, the static image of the picked-up image may be stored in a static image storage server (not shown), which is connected to the network, instead of or in addition to the static image storage unit 42.

A static image-acquisition instruction may be given using an operation device other than the freeze switch 13b. For example, a foot pedal may be connected to the processor device 16, and a static image-acquisition instruction may be given in a case where a user operates the foot pedal (not shown) with a foot. The foot pedal may also be used to switch a mode. Further, a static image-acquisition instruction may be given or a mode may be switched by the input of voice, the input of a sight line, the input of a gesture, or the like.

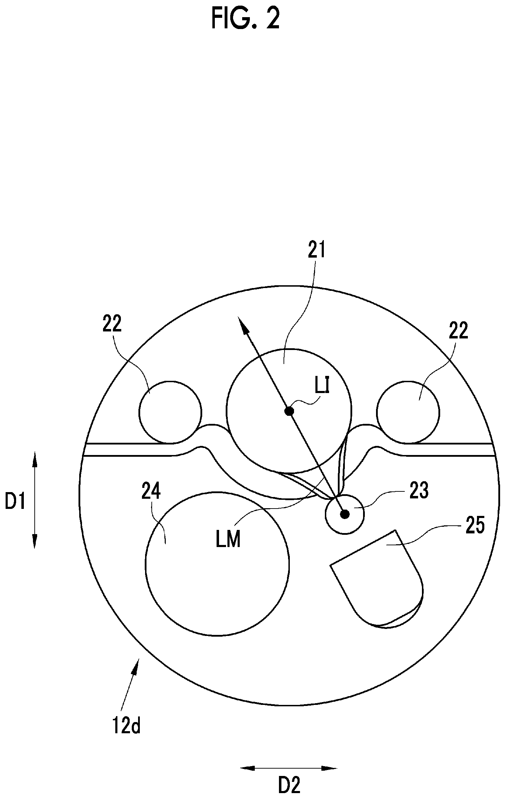

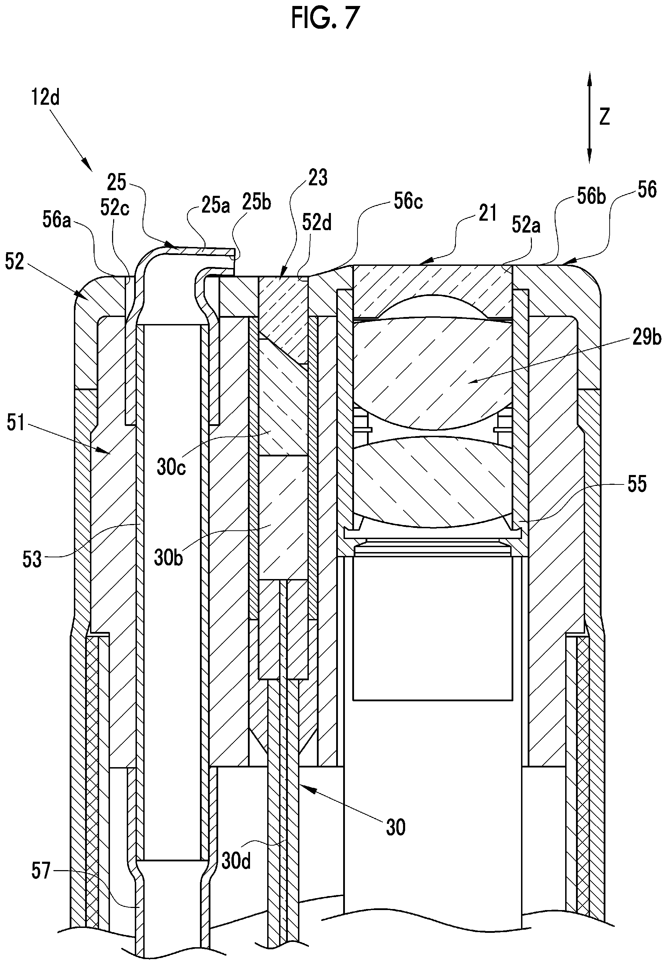

As shown in Fig. 2, the distal end part of the endoscope 12 has a substantially circular shape; and is provided with an objective lens 21 that is positioned closest to a subject among optical members of an image pickup optical system 29b (see Fig. 7) of the endoscope 12, two illumination lenses 22 that are used to irradiate a subject with illumination light, a lens 23 for auxiliary measurement light that is used to illuminate a subject with the auxiliary measurement light to be described later, a treatment tool outlet 24 that allows a treatment tool to protrude toward a subject, and an air/water supply nozzle 25 that is used to supply air and water. The objective lens 21 forms an observation window disclosed in claims. The air/water supply nozzle 25 corresponds to a fluid jet nozzle disclosed in claims. Liquid to be supplied by the air/water supply nozzle 25 is washing water, and gas to be supplied is air or carbon dioxide.

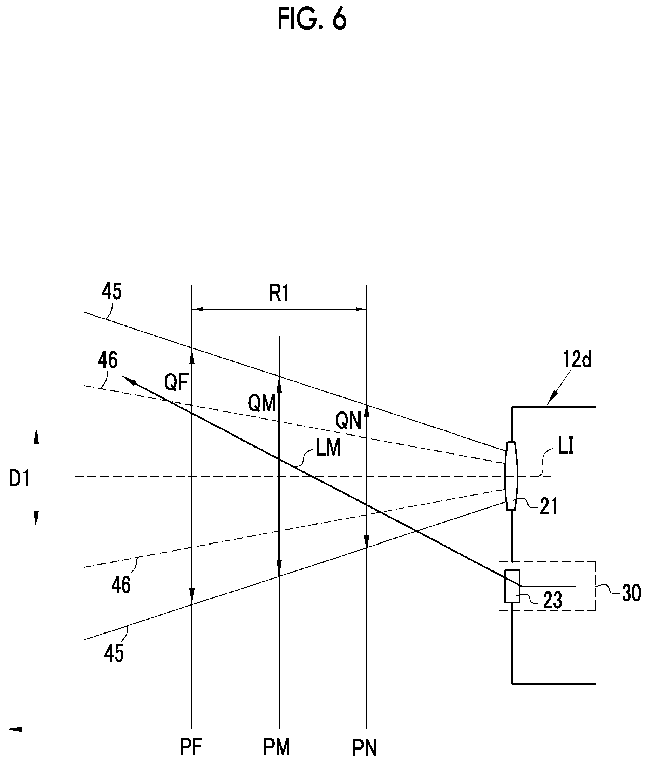

An optical axis LI (see Fig. 6) of the objective lens 21 extends in a direction perpendicular to the plane of paper. A vertical first direction D1 is orthogonal to the optical axis LI, and a horizontal second direction D2 is orthogonal to the optical axis LI and the first direction D1.

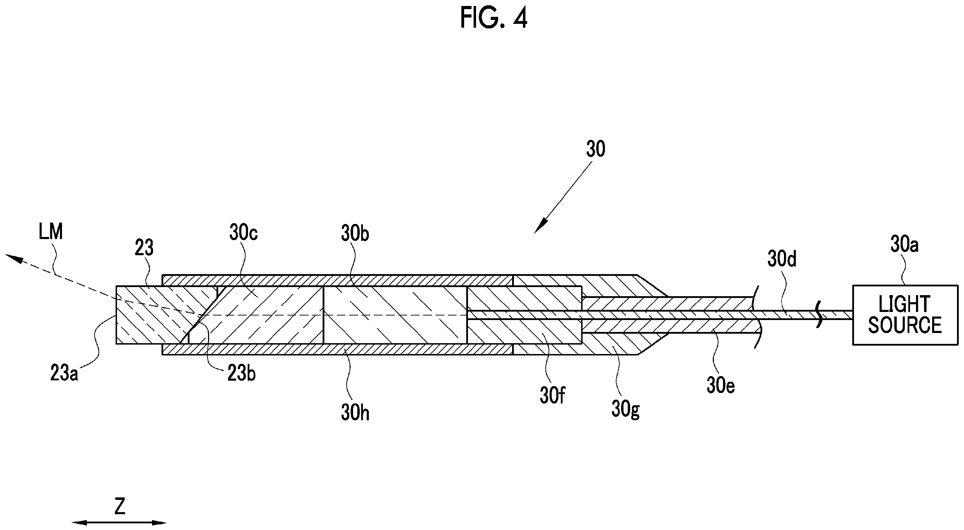

As shown in Fig. 3, the light source device 14 comprises a light source unit 26 and a light source controller 27. The light source unit 26 (illumination light source unit) generates illumination light that is used to illuminate a subject. The illumination light emitted from the light source unit 26 is incident on a light guide 28, and a subject is irradiated with the illumination light through the illumination lenses 22. It is preferable that a white light source emitting white light, a plurality of light sources, which include a white light source and a light source emitting another color light (for example, a blue light source emitting blue light), or the like is used as a light source of the illumination light in the light source unit 26. The light source controller 27 is connected to a system controller 41 of the processor device 16. The light source controller 27 controls the light source unit 26 on the basis of an instruction given from the system controller 41. The system controller 41 (light emitting controller) not only gives an instruction related to light source control to the light source controller 27 but also controls a light source 30a (see Fig. 4) of an auxiliary measurement light-emitting unit 30. The details of the light source control performed by the system controller 41 will be described later.

The distal end part 12d of the endoscope 12 is provided with an illumination optical system 29a, an image pickup optical system 29b, and an auxiliary measurement light-emitting unit 30. The illumination optical system 29a includes the illumination lenses 22, and an object to be observed is irradiated with light, which is emitted from the light guide 28, through the illumination lenses 22. The image pickup optical system 29b includes the objective lens 21 and an image pickup element 32. Light reflected from the object to be observed is incident on the image pickup element 32 through the objective lens 21. Accordingly, the reflected image of the object to be observed is formed on the image pickup element 32.

The image pickup element 32 is a color image pickup sensor, and picks up the reflected image of an object to be examined and outputs image signals. It is preferable that the image pickup element 32 is a charge coupled device (CCD) image pickup sensor, a complementary metal-oxide semiconductor (CMOS) image pickup sensor, or the like. The image pickup element 32 used in the embodiment of the present invention is a color image pickup sensor that is used to obtain RGB image signals corresponding to three colors of R (red), G (green), and B (blue). The image pickup element 32 is controlled by an image pickup controller 33. A complementary color image pickup element, which is provided with color filters corresponding to complementary colors, that is, C (cyan), M (magenta), Y (yellow), and G (green), may be used as the image pickup element 32.

The image signals output from the image pickup element 32 are transmitted to a CDS/AGC circuit 34. The CDS/AGC circuit 34 performs correlated double sampling (CDS) or auto gain control (AGC) on the image signals that are analog signals. The image signals, which have been transmitted through the CDS/AGC circuit 34, are converted into digital image signals by an analog/digital converter (A/D converter) 35. The digital image signals, which have been subjected to A/D conversion, are input to the processor device 16 through a communication interface (I/F) 36.

The processor device 16 comprises a communication interface (I/F) 38 that is connected to the communication I/F 36 of the endoscope 12, a signal processing unit 39, a display controller 40, and the system controller 41. The communication I/F 38 receives the image signals, which are transmitted from the communication I/F 36 of the endoscope 12, and transmits the image signals to the signal processing unit 39. A memory, which temporarily stores the image signals received from the communication I/F 38, is built in the signal processing unit 39, and the signal processing unit 39 processes an image signal group, which is a set of the image signals stored in the memory, to generate the picked-up image.

The signal processing unit 39 acquires an image picked up by the endoscope. In a case where the endoscope 12 is set to the length measurement mode, the signal processing unit 39 may be adapted to perform structure-emphasis processing of emphasizing structures, such as blood vessels, or color difference-emphasis processing of increasing a color difference between a normal area and a lesion area of the object to be observed on the picked-up image.

The display controller 40 causes the monitor 18 to display the picked-up image that is generated by the signal processing unit 39. The system controller 41 performs the control of the image pickup element 32 through the image pickup controller 33 provided in the endoscope 12. The image pickup controller 33 also performs the control of the CDS/AGC 34 and the A/D 35 together with the control of the image pickup element 32. A static image storage controller 43 performs a control related to the static image of the picked-up image to be stored in the static image storage unit 42. The static image storage controller 43 performs a control to be described later in a case where a static image-acquisition instruction is given once in the length measurement mode.

As shown in Fig. 4, the auxiliary measurement light-emitting unit 30 (auxiliary measurement light source unit) comprises a light source 30a, a Gradient Index (GRIN) lens 30b, a prism 30c, an optical fiber 30d, and the lens 23 for auxiliary measurement light. The light source 30a is to emit light having a color that can be detected by pixels of the image pickup element 32 (specifically visible light), and includes a light-emitting element, such as a laser light source LD (laser diode) or a light emitting diode (LED), and a condenser lens that condenses light emitted from the light-emitting element. The lens 23 for auxiliary measurement light forms an auxiliary light-emitting window and an optical member for the auxiliary light-emitting window disclosed in claims. The lens 23 for auxiliary measurement light is formed in a columnar shape. In a case where the lens 23 for auxiliary measurement light is assembled in the distal end part 12d, a distal end surface 23a of the lens 23 for auxiliary measurement light is a flat surface that is orthogonal to an axial direction Z of the insertion part 12a and a proximal end surface 23b thereof is a flat surface that intersects with the axial direction Z and mates with the distal end surface of the prism 30c.

It is preferable that the wavelength of the light emitted from the light source 30a is, for example, 600 nm or more and 750 nm or less, it is more preferable that the wavelength of the light emitted from the light source 30a is 600 nm or more and 700 nm or less, and it is most preferable that the light emitted from the light source 30a is red light having a wavelength of 630 nm or more and 660 nm or less. Alternatively, green light having a wavelength of 495 nm or more and 570 nm or less may be used. The light source 30a is controlled by the system controller 41 and emits light on the basis of an instruction given from the system controller 41.

The proximal end side (light source 30a side) of the optical fiber 30d is covered with a fiber sheath 30e, the distal end side (a side that emits laser light) thereof is inserted into a ferrule 30f and adhered to the ferrule 30f by an adhesive, and the end surfaces thereof are polished.

The GRIN lens 30b is mounted on the distal end side of the ferrule 30f and the prism 30c is mounted on the distal end side of the GRIN lens 30b, so that an assembly is formed. The ferrule 30f is a member that is used to hold and connect the optical fiber 30d, and a hole into which the optical fiber 30d is to be inserted penetrates the central portion of the ferrule 30f in the axial direction (a horizontal direction in Fig. 4).

A reinforcing material 30g is provided on the outside of the ferrule 30f and the fiber sheath 30e to protect the optical fiber 30d and the like. The lens 23 for auxiliary measurement light, the GRIN lens 30b, and the prism 30c are housed in a housing 30h. The housing 30h is joined to the reinforcing material 30g. Accordingly, the lens 23 for auxiliary measurement light, the GRIN lens 30b, the prism 30c, and the optical fiber 30d are integrally held in the reinforcing material 30g and the housing 30h.

The optical fiber 30d guides light, which is emitted from the light source 30a, to the GRIN lens 30b. The GRIN lens 30b is to convert the light, which is guided from the light source 30a by the optical fiber 30d, into highly coherent light again to convert the light, which is emitted from the light source 30a, into auxiliary measurement light that is used for the measurement of a subject.

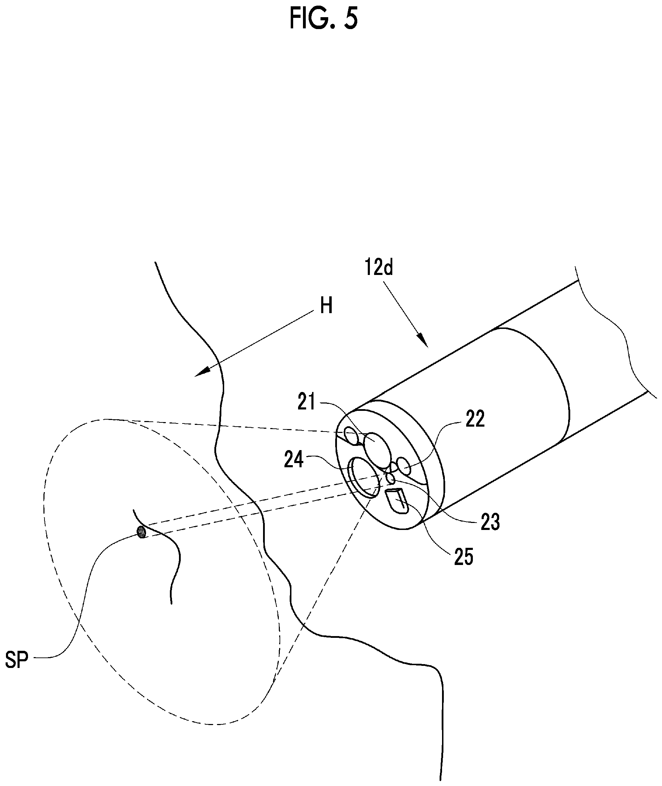

The prism 30c is an optical member that is used to change the travel direction of the auxiliary measurement light converted by the GRIN lens 30b. The prism 30c changes the travel direction of the auxiliary measurement light so that the auxiliary measurement light intersects with the visual field of the image pickup optical system 29b including the objective lens 21 and lens groups. The details of the travel direction of the auxiliary measurement light will also be described later. A subject is irradiated with the auxiliary measurement light, which is emitted from the prism 30c, through the lens 23 for auxiliary measurement light. In a case where a subject H is irradiated with the auxiliary measurement light as shown in Fig. 5, a spot SP as a circular region (specific region) is formed on the subject.

An auxiliary measurement slit, which is formed at the distal end part 12d of the endoscope, may be used instead of the lens 23 for auxiliary measurement light. Further, it is preferable that an anti-reflection coating (AR coating) (anti-reflection portion) is provided on the lens 23 for auxiliary measurement light. The reason why the anti-reflection coating is provided as described above is that it is difficult for the signal processing unit 39 to recognize the position of the spot SP formed on the subject by the auxiliary measurement light in a case where the auxiliary measurement light is reflected without being transmitted through the lens 23 for auxiliary measurement light and a ratio of the auxiliary measurement light with which a subject is irradiated is reduced.

The auxiliary measurement light-emitting unit 30 has only to be capable of emitting the auxiliary measurement light to the visual field of the image pickup optical system. For example, the light source 30a may be provided in the light source device and light emitted from the light source 30a may be guided to the GRIN lens 30b by the optical fiber 30d. Further, the prism 30c may not be used, a diffractive optical element (DOE) may be used instead of the GRIN lens 30b, and the directions of the light source 30a, the DOE, and an optical fiber, which guides light emitted from the light source 30a to the DOE, may be inclined with respect to the optical axis LI so that the auxiliary measurement light is emitted in a direction crossing the visual field of the image pickup optical system.

With regard to the travel direction of the auxiliary measurement light, the auxiliary measurement light is emitted in a state where an optical axis LM of the auxiliary measurement light intersects with the optical axis LI of the objective lens 21 as shown in Fig. 6. In a case where a subject can be observed in a range R1 of an observation distance, it is understood that the positions (points where the respective arrows QN, QM, and QF intersect with the optical axis LM) of the spot SP, which is formed on the subject by the auxiliary measurement light, in image pickup ranges (shown by arrows QN, QM, and QF) at a near end PN, an intermediate vicinity PM, and a far end PF of the range R1 are different from each other. The image pickup angle of view of the image pickup optical system is represented by a region between two solid lines 45, and measurement is performed in a central region (a region between two dotted lines 46), in which an aberration is small, of this image pickup angle of view.

Since the auxiliary measurement light is emitted in a state where the optical axis LM of the auxiliary measurement light intersects with the optical axis LI as described above, sensitivity to the movement of the position of the spot with respect to a change in the observation distance is high. Accordingly, the size of the subject can be measured with high accuracy. Then, the image of the subject illuminated with the auxiliary measurement light is picked up by the image pickup element 32, so that a picked-up image including the spot SP is obtained.

The signal processing unit 39 functions as a position specifying section that specifies the position of the spot SP on the basis of the picked-up image. Specifically, the signal processing unit 39 specifies coordinate information about the position of the spot SP. The spot SP is displayed as a substantially circular red region, which includes a large amount of components corresponding to the color of the auxiliary measurement light, in the picked-up image. Accordingly, the signal processing unit 39 specifies the position of the spot SP from the substantially circular red region. As a method of specifying a position, for example, a picked-up image is binarized and the centroid of a white portion (pixel at which the signal intensity is higher than a threshold value for binarization) of a binarized image is specified as the position of the spot SP.

The signal processing unit 39 also functions as an observation distance detection unit that detects an observation distance on the basis of the position of the spot SP. The signal processing unit 39 detects an observation distance from the position of the spot SP with reference to an observation distance table in which a relationship between the position of the spot SP in the picked-up image and an observation distance is stored. It is preferable that coordinate information about the position of the spot SP, an observation distance, and the like are stored as additional data of the picked-up image.

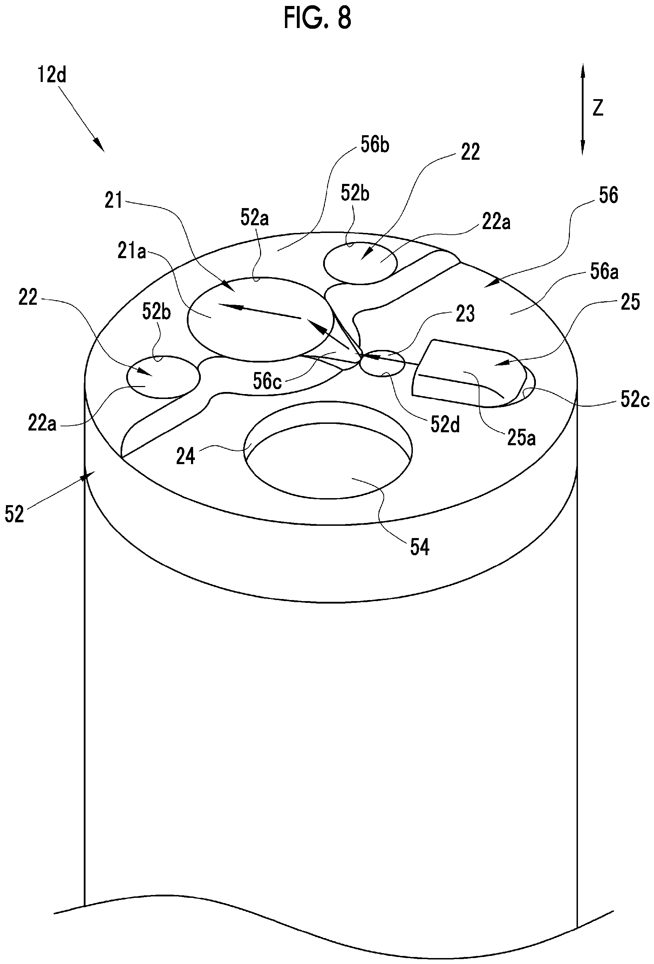

As shown in Fig. 7, the distal end part 12d comprises a distal end part body 51, a distal end cap 52, the objective lens 21, the illumination lenses 22 (see Fig. 8), the treatment tool outlet 24 (see Fig. 8), the air/water supply nozzle 25, and the like. The distal end part body 51 is made of a hard material, such as metal, and holds the respective components, which are disposed in the distal end part 12d, such as the image pickup optical system 29b, the air/water supply nozzle 25, a connection pipe 53, the light guide 28 (see Fig. 3), and a treatment tool-insertion tube 54 (see Fig. 8). In Fig. 7, the light guide 28, the treatment tool-insertion tube 54, and the like are not shown in order to prevent the drawings from becoming complicated.

The distal end cap 52 is made of an insulating resin material, and covers the distal end side of the distal end part body 51 in an axial direction Z of the insertion part 12a. In the following description, an end surface positioned on the distal end side (objective side) in the axial direction Z may be referred to as a distal end surface or a distal end, and an end surface positioned on a side opposite to the objective side may be referred to as a proximal end surface or a proximal end.

Through-holes 52a to 52d through which the objective lens 21, the illumination lenses 22, the air/water supply nozzle 25, and the lens 23 for auxiliary measurement light are exposed and the treatment tool outlet 24 (see Fig. 8) are formed in the distal end cap 52. The objective lens 21 also serves as a cover glass for the image pickup optical system 29b, and is a lens that is positioned closest to the distal end side of the image pickup optical system 29b. The image pickup optical system 29b including the objective lens 21 is held by a lens barrel 55. The lens barrel 55 holds the proximal end side of the outer peripheral surface of the objective lens 21. The distal end side of the outer peripheral surface of the objective lens 21 is fitted to the through-hole 52a of the distal end cap 52.

The lens barrel 55 is held by the distal end part body 51. The distal end surface of the lens barrel 55 abuts on the proximal end side of the distal end cap 52, and the objective lens 21 is disposed at a position where the objective lens 21 is exposed from the distal end side of the distal end cap 52. The objective lens 21 may be a cover glass that is positioned closest to the distal end side of the image pickup optical system 29b and does not have a lens effect. Further, the objective lens 21 may not be a component of the image pickup optical system 29b, and may be a component that is fitted and fixed to the through-hole 52d of the distal end cap 52 as a mere cover glass.

Furthermore, the treatment tool outlet 24 communicates with a treatment tool inlet port 12f (see Fig. 1) of the operation part 12b through the treatment tool-insertion tube 54 inserted into the insertion part 12a, and a treatment tool inserted from the treatment tool inlet port 12f is led to the treatment tool outlet 24.

A suction tube (not shown) is connected to the treatment tool-insertion tube 54, and suction from the treatment tool outlet 24 is performed by the operation of a suction button 12g (see Fig. 1) of the operation part 12b.

As shown in Fig. 8, the distal end cap 52 is provided with a distal end surface 56. The distal end surface 56 includes a flat surface 56a, a flat surface 56b, and a guide surface 56c. The flat surface 56a is a flat surface orthogonal to the axial direction Z. The flat surface 56b is parallel to the flat surface 56a, and is positioned closer to the distal end side than the flat surface 56a in the axial direction Z. The guide surface 56c is disposed between the flat surface 56a and the flat surface 56b.

The above-mentioned through-holes 52a and 52b are disposed on the flat surface 56b. That is, a distal end surface 21a of the objective lens 21 and distal end surfaces 22a of a pair of illumination lenses 22, which are exposed from the through-holes 52a and 52b, are disposed on the flat surface 56b. The objective lens 21 is disposed between the pair of illumination lenses 22. The distal end surface 21a of the objective lens 21 and the distal end surfaces 22a of the illumination lenses 22 are flat surfaces, and are disposed on the same plane as the flat surface 56b in the axial direction Z (also see Fig. 7).

The above-mentioned through-holes 52c and 52d are disposed on the flat surface 56a. The air/water supply nozzle 25 is exposed from the through-hole 52c. That is, the flat surface 56a is a mounting position for the air/water supply nozzle 25 in the axial direction Z. The air/water supply nozzle 25 is connected to an air/liquid supply tube 57 through the connection pipe 53. The proximal end portion of the air/water supply nozzle 25 is fitted to the outside of one end portion of the connection pipe 53, and one end portion of the air/liquid supply tube 57 is fitted to the outside of the other end portion of the connection pipe 53.

A jetting cylinder portion 25a is formed on the distal end side of the air/water supply nozzle 25. The jetting cylinder portion 25a is formed in the shape of a cylinder that protrudes in a direction bent by, for example, 90° from the proximal end portion of the air/water supply nozzle 25, and includes a jetting port 25b at the distal end thereof. The jetting cylinder portion 25a is disposed to protrude from the through-hole 52c to the distal end side in the axial direction Z.

The jetting port 25b of the air/water supply nozzle 25 is disposed to face the objective lens 21. Accordingly, the air/water supply nozzle 25 jets a washing solution or gas, which is fluid, to the distal end surface 21a of the objective lens 21 and the peripheral portion of the distal end surface 21a.

The air/water supply nozzle 25 communicates with the air/liquid supply tube 57 inserted into the endoscope 12, and is connected to an air/water supply device (not shown), which is connected to the endoscope 12, through the air/liquid supply tube 57.

Then, in a case where a leak hole formed in an air/water supply button 12h (see Fig. 1) of the operation part 12b is closed with a finger, gas supplied from the air/water supply device is jetted from the air/water supply nozzle 25. In a case where the air/water supply button 12h is pressed with the finger closing the leak hole, a washing solution supplied from the air/water supply device is jetted from the air/water supply button 12h.

As a procedure for washing the objective lens 21, for example, a washing solution is jetted from the air/water supply nozzle 25 to remove adhering materials, which adhere to the objective lens 21, such as blood and body fluid, and gas is then jetted from the air/water supply nozzle 25 to remove the washing solution that remains on the objective lens 21 or a region adjacent to the objective lens 21.

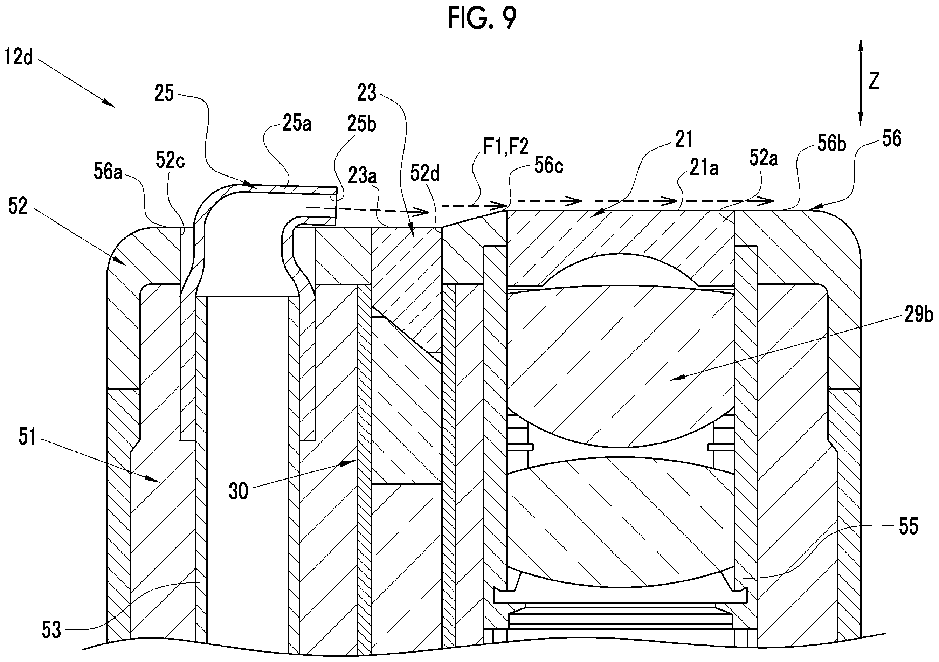

It is preferable that, in a case where washing water or gas is jetted toward the objective lens 21 from the air/water supply nozzle 25 as shown in Fig. 9, the flow velocity F1 of the washing water at a position where the washing water or gas reaches the objective lens 21, that is, the outer peripheral edge of the objective lens 21 is 2 m/s or more and the flow velocity F2 of the gas at the outer peripheral edge of the objective lens 21 is 40 m/s or more. It is preferable that the flow velocities F1 and F2 satisfy the above-mentioned values regardless of the orientation of the distal end part 12d. For example, in a case where the air/water supply nozzle 25 is positioned immediately below the objective lens 21 in a vertical direction, the flow velocity of washing water or gas is reduced due to an influence of gravity but it is preferable that the flow velocities F1 and F2 satisfy the above-mentioned values even in this case.

The distal end surface 23a of the lens 23 for auxiliary measurement light, which is exposed from the through-hole 52d, is disposed on the flat surface 56a. That is, the mounting position for the air/water supply nozzle 25 and the distal end surface 23a of the lens 23 for auxiliary measurement light are disposed at the same position in the axial direction Z. The lens 23 for auxiliary measurement light is disposed between the objective lens 21 and the air/water supply nozzle 25 in the fluid jet range of the air/water supply nozzle 25.

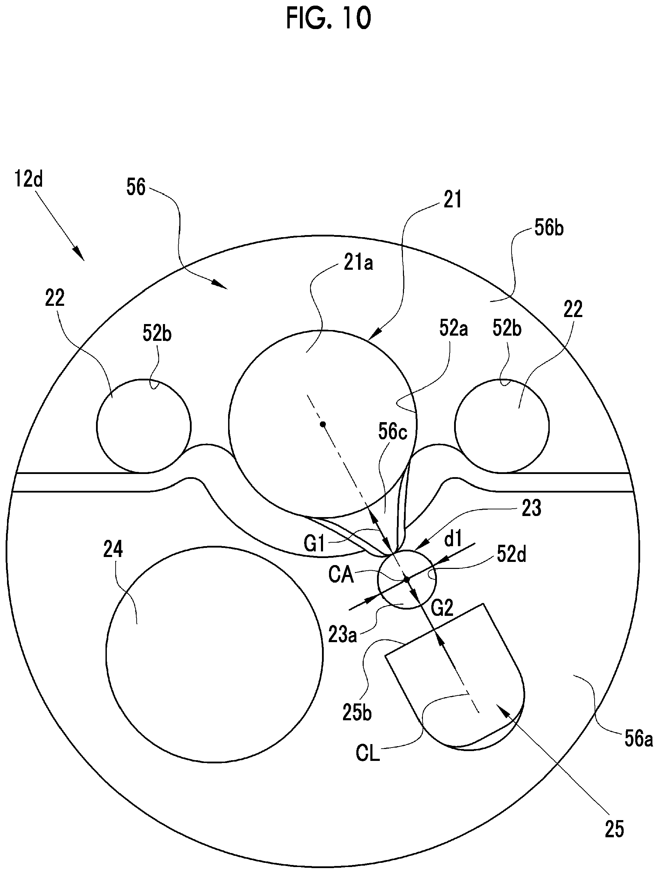

As shown in Fig. 10, in this embodiment, the lens 23 for auxiliary measurement light is disposed in a region, which connects the jetting port 25b of the air/water supply nozzle 25 to the outer peripheral edge of the objective lens 21, in a case where the distal end surface 56 is viewed in the axial direction Z. Accordingly, in a case where fluid is jetted to the objective lens 21 from the air/water supply nozzle 25, fluid can be jetted to even the lens 23 for auxiliary measurement light at the same time.

Further, a central axis CA of the lens 23 for auxiliary measurement light is positioned on a center line CL of the jetting cylinder portion 25a in this embodiment, but the present invention is not limited thereto. The lens 23 for auxiliary measurement light has only to be disposed between the objective lens 21 and the air/water supply nozzle 25 in the fluid jet range of the air/water supply nozzle 25 and the position of the central axis CAmay be shifted from the center line CL.

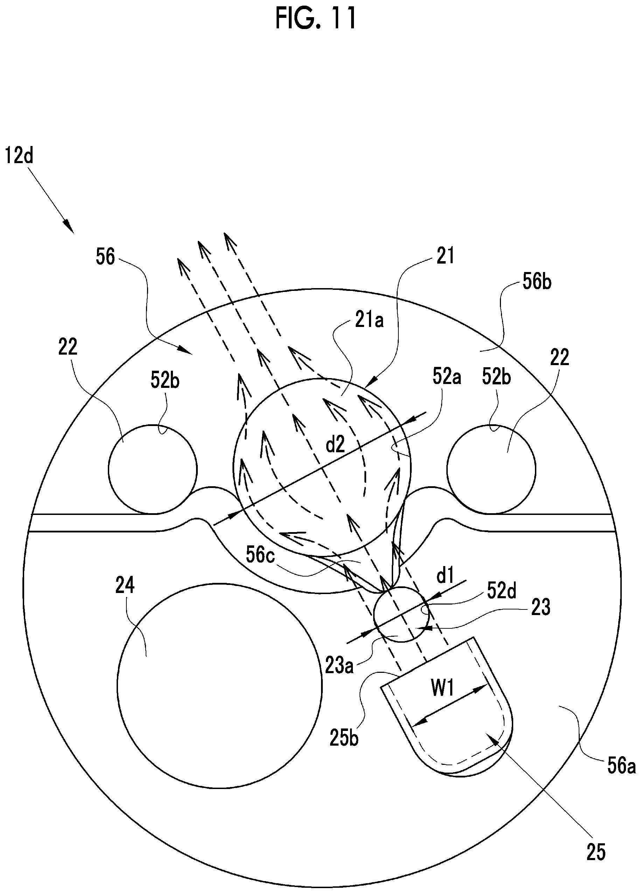

It is preferable that an outer diameter d1 of the lens 23 for auxiliary measurement light is 0.5 mm or more and 1.6 mm or less, a first minimum distance G1, which is the minimum distance between the outer peripheral edge of the objective lens 21 and the outer peripheral edge of the lens 23 for auxiliary measurement light, is 0 mm or more and 1.5 mm or less, and a second minimum distance G2, which is the minimum distance between the outer peripheral edge of the lens 23 for auxiliary measurement light and the distal end of the air/water supply nozzle 25, is 0 mm or more and 0.5 mm or less. In a case where the first minimum distance G1 and the second minimum distance G2 are set to be as small as possible, the washing water or gas is blown to the objective lens 21 while maintaining a sufficient flow velocity at a time when the washing water or gas jetted toward the objective lens 21 from the air/water supply nozzle 25 reaches the objective lens 21. Further, it is preferable that the outer diameter d1 of the lens 23 for auxiliary measurement light is smaller than the outer diameter d2 (see Fig. 11) of the objective lens 21. Furthermore, it is preferable that the outer diameter d1 of the lens 23 for auxiliary measurement light is smaller than an opening width W1 (see Fig. 11) of the air/water supply nozzle 25.

In this embodiment, the guide surface 56c is provided between the flat surface 56a and the flat surface 56b. The flat surface 56a and the flat surface 56b have a level difference therebetween in the axial direction Z as described above, and the guide surface 56c is formed of a continuous surface that connects the flat surface 56a to the flat surface 56b. Specifically, the guide surface 56c is an inclined surface that is formed in a flat shape from a position where the guide surface 56c is in contact with the outer peripheral edge of the lens 23 for auxiliary measurement light to a position where the guide surface 56c is in contact with the outer peripheral edge of the objective lens 21.

Since the guide surface 56c is disposed in the fluid jet range of the air/water supply nozzle 25, fluid is jetted to even the guide surface 56c in a case where fluid is jetted from the air/water supply nozzle 25. The fluid jetted to the guide surface 56c is diffused and blown to the objective lens 21. In this case, the entire guide surface 56c may be included in the fluid jet range of the air/water supply nozzle 25 or only a part of the guide surface 56c may be included in the fluid jet range of the air/water supply nozzle 25. In this embodiment, the entire guide surface 56c is included in a region that connects the jetting port 25b of the air/water supply nozzle 25 to the outer peripheral edge of the objective lens 21.

As shown in Fig. 11, the opening width W1 of the air/water supply nozzle 25 is smaller than the outer diameter d2 of the objective lens 21. Since the guide surface 56c is positioned in the fluid jet range of the air/water supply nozzle 25 as described above, fluid is diffused by the guide surface 56c. Accordingly, since the fluid diffused by the guide surface 56c is blown to the objective lens 21 (a state shown by a broken line arrow of Fig. 11), it is possible to improve the washability of the objective lens 21 of which the outer diameter d2 is larger than the opening width W1 of the air/water supply nozzle 25.

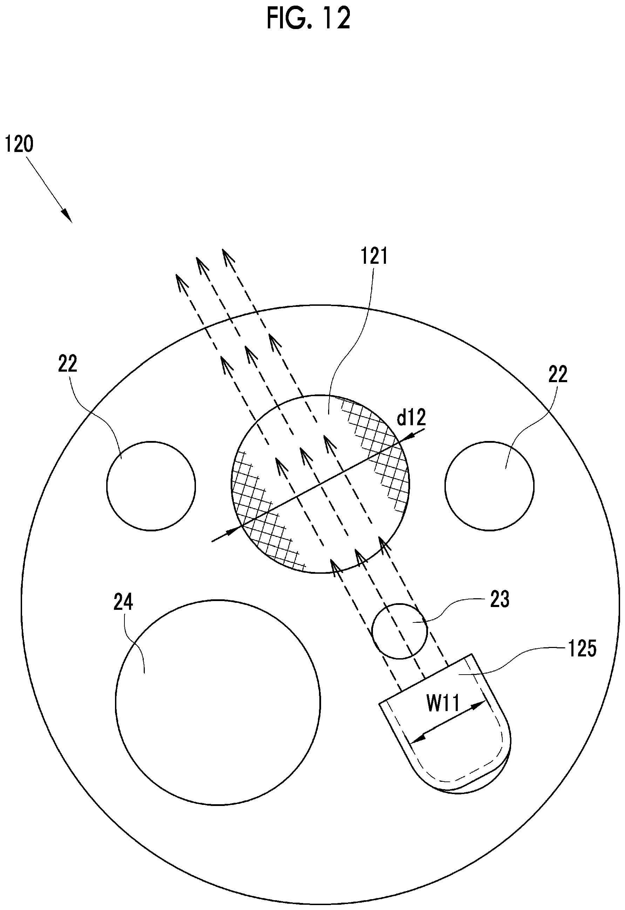

If the guide surface 56c is not provided, fluid jetted from an air/water supply nozzle 125 goes straight as it is without being diffused on a distal end part 120 and is blown to an objective lens 121 as shown in a comparative example of Fig. 12. Accordingly, fluid may not be blown to portions (portions shown by hatching), which are positioned on both sides of a fluid jet range, of the obj ective lens 121 of which an outer diameter d12 is larger than an opening width W11 of the air/water supply nozzle 125, and dirt may remain thereon. On the other hand, since the guide surface 56c is provided in this embodiment, this does not occur.

The action of the configuration will be described. In a case where the endoscope 12 is set to the length measurement mode, the system controller 41 performs a control to operate the image pickup element 32 through the image pickup controller 33 and performs a control to operate the light source unit 26 of the light source device 14 and the light source 30a of the auxiliary measurement light-emitting unit 30, and controls the illumination light and the auxiliary measurement light according to a preset light emission pattern. Then, after the image pickup element 32, the light source unit 26, and the light source 30a starts to be operated, the insertion part 12a is inserted into an object to be examined, for example, the alimentary canal.

A portion to be observed present in the alimentary canal is irradiated with light, which is emitted from the light source device 14, through the light guide 28 and the illumination lenses 22. The portion to be observed is irradiated with the auxiliary measurement light, which is emitted from the light source 30a, through the lens 23 for auxiliary measurement light. The image pickup element 32 picks up the inside of the alimentary canal and outputs image pickup signals. The image pickup signals are input to the processor device 16 through the communication I/F 36 and the communication I/F 38, and are displayed on the monitor 18. The auxiliary measurement light is applied, so that the spot SP appears in a picked-up image.

In a case where dirt adheres to the objective lens 21 or a case where dirt adheres to the lens 23 for auxiliary measurement light, washing water is jetted from the jetting port 25b by the operation of the air/water supply button 12h and washes the objective lens 21. After the objective lens 21 is washed, gas is further jetted from the jetting port 25b by the operation of the air/water supply button 12h to blow off the washing water remaining on the objective lens 21.

Since the lens 23 for auxiliary measurement light is disposed between the objective lens 21 and the air/water supply nozzle 25 in the fluid jet range of the air/water supply nozzle 25 as described above, fluid can be jetted to even the lens 23 for auxiliary measurement light at the same time in a case where fluid is jetted to the objective lens 21 from the air/water supply nozzle 25. Accordingly, since the lens 23 for auxiliary measurement light can be washed as well as the objective lens 21 at the same time, the dirt of the lens 23 for auxiliary measurement light can be efficiently removed.

In addition, in a case where washing water or gas is jetted toward the objective lens 21 from the air/water supply nozzle 25, the flow velocity F1 of the washing water at the outer peripheral edge of the objective lens 21 is set to 2 m/s or more and the flow velocity F2 of the gas at the outer peripheral edge of the objective lens 21 is set to 40 m/s or more. Accordingly, fluid is jetted at a flow velocity sufficient to remove the dirt of the lens 23 for auxiliary measurement light while removing the dirt of the objective lens 21.

### [Second embodiment]

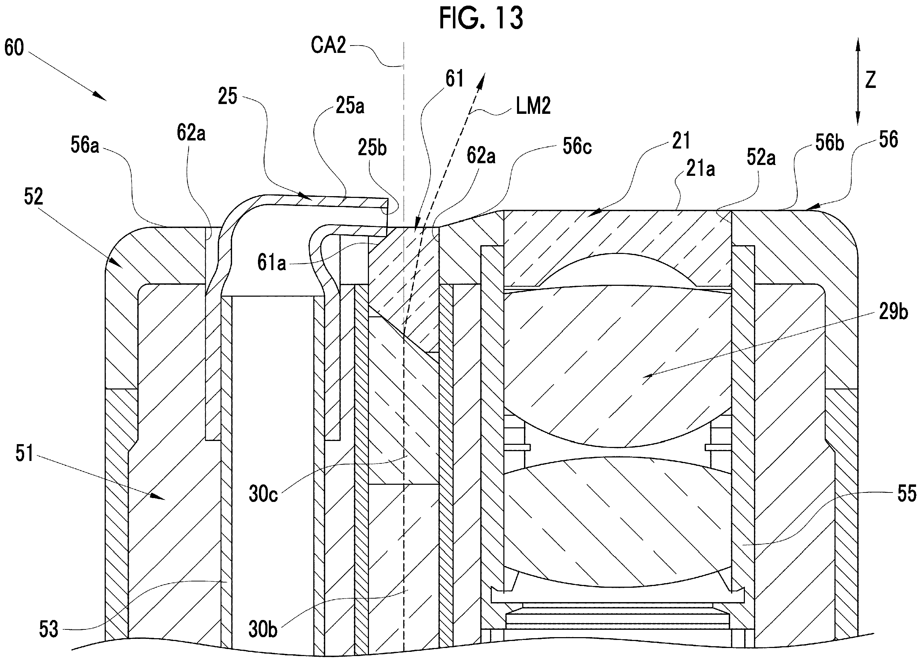

In the first embodiment, an interval of the second minimum distance G2 is formed between the outer peripheral edge of the lens 23 for auxiliary measurement light and the distal end of the air/water supply nozzle 25. However, in a second embodiment, this interval is set to 0 mm and a lens for auxiliary measurement light and an air/water supply nozzle are caused to be in contact with each other so that the position of the lens for auxiliary measurement light is restricted. In a distal end part 60 shown in Fig. 13, a lens 61 for auxiliary measurement light includes a notched portion 61a. The notched portion 61a is disposed at a position facing the air/water supply nozzle 25. Since components other than the lens 61 for auxiliary measurement light and through-holes 62a and 62b of a distal end cap 52 are the same as those of the distal end part 12d and the auxiliary measurement light-emitting unit 30 of the first embodiment, these components will be denoted by the same reference numerals and the description thereof will be omitted.

The notched portion 61a is formed at a position that avoids the optical path of auxiliary measurement light emitted from the lens 61 for auxiliary measurement light. In this embodiment, the notched portion 61a is disposed at a position opposite to an optical axis LM2 of the auxiliary measurement light with respect to a central axis CA2 of the lens 61 for auxiliary measurement light. Accordingly, the outer shape of a spot SP, which is formed on a subject in a case where the subject is irradiated with the auxiliary measurement light, is not lost.

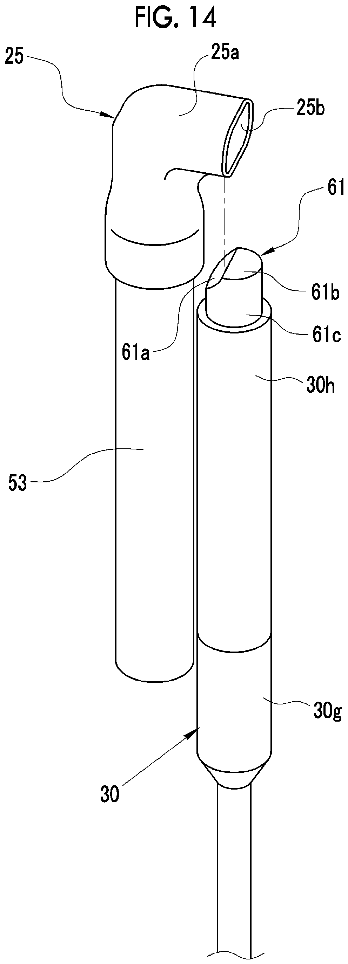

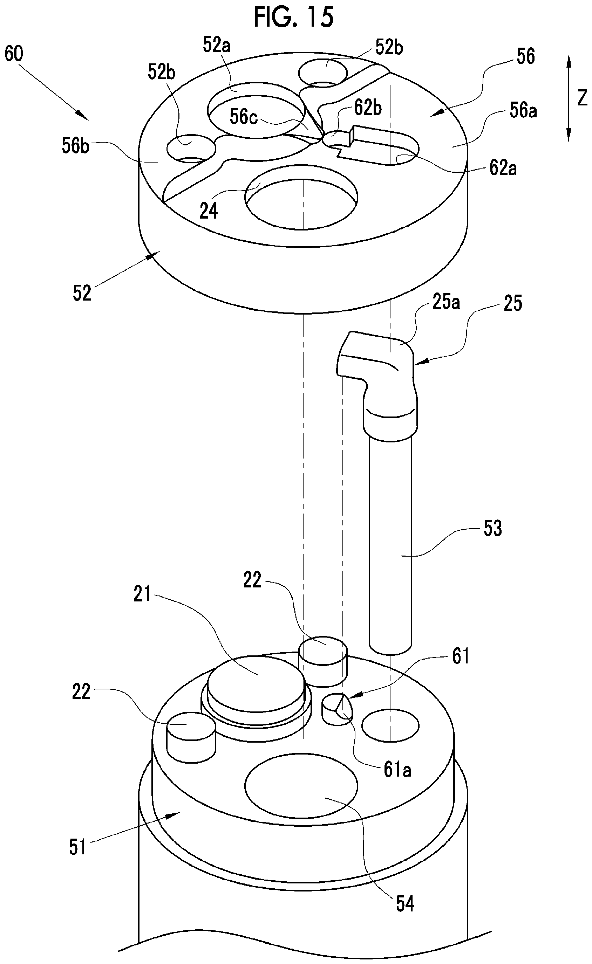

As shown in Fig. 14, the lens 61 for auxiliary measurement light is formed in a columnar shape like the lens 23 for auxiliary measurement light of the first embodiment but is different from the lens 23 for auxiliary measurement light in that the lens 61 for auxiliary measurement light includes the notched portion 61a. The notched portion 61a is an inclined surface that is inclined toward an outer peripheral surface 61c from a distal end surface 61b of the lens 61 for auxiliary measurement light. The air/water supply nozzle 25 is assembled in the distal end part 60 in a state where the distal end of the jetting cylinder portion 25a is in contact with the notched portion 61a. The air/water supply nozzle 25 and the lens 61 for auxiliary measurement light are held by the distal end part body 51 as in the first embodiment. In this case, the air/water supply nozzle 25 and the lens 61 for auxiliary measurement light are assembled in the distal end part 60 in a state where the air/water supply nozzle 25 and the lens 61 for auxiliary measurement light are in contact with each other. Accordingly, through-holes 62a and 62b (see Fig. 15) of the distal end cap 52, which are provided to allow the air/water supply nozzle 25 and the lens 61 for auxiliary measurement light to be exposed, are formed integrally.

In a step of assembling the distal end part 60, for example, as shown in Fig. 15, the air/water supply nozzle 25 is held together with the connection pipe 53 by the distal end part body 51 after the lens 61 for auxiliary measurement light is held together with the prism 30c, the housing 30h, and the like by the distal end part body 51. Since the air/water supply nozzle 25 is assembled in a state where the jetting cylinder portion 25a of the air/water supply nozzle 25 and the notched portion 61a are in contact with each other as described above, the jetting cylinder portion 25a of the air/water supply nozzle 25 to be in contact with the notched portion 61a is positioned closer to the distal end side in the axial direction Z than the lens 61 for auxiliary measurement light. Accordingly, the position of the lens 61 for auxiliary measurement light in the axial direction Z is restricted.

Since the position of the lens 61 for auxiliary measurement light is restricted by the air/water supply nozzle 25 as described above, the lens 61 for auxiliary measurement light can be accurately positioned in the axial direction Z. Further, since the position of the lens 61 for auxiliary measurement light is restricted by the air/water supply nozzle 25, the separation of the lens 61 for auxiliary measurement light in the axial direction Z can be prevented. Furthermore, since the distal end side of the lens 61 for auxiliary measurement light does not need to be fixed to the distal end part body 51 by adhesion or the like in the assembly step, the number of steps of assembling the distal end part 60 can also be reduced.

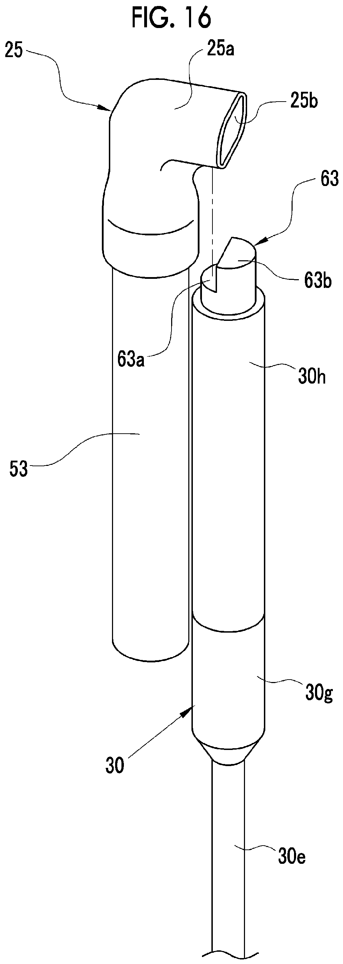

Moreover, an inclined surface, which is inclined toward the outer peripheral surface 61c from the distal end surface 61b of the lens 61 for auxiliary measurement light, is formed on the lens 61 for auxiliary measurement light as the notched portion 61a in the second embodiment, but the present invention is not limited thereto. A notched portion, which faces the air/water supply nozzle 25 and is in contact with the air/water supply nozzle 25 in a case where the lens 61 for auxiliary measurement light is assembled in the distal end part 60, may be formed or, for example, a notched portion 63a having a level difference, which is recessed from a distal end surface 63b of a lens 63 for auxiliary measurement light and is parallel to the distal end surface 63b, may be formed as shown in Fig. 16. Accordingly, the position of the lens 63 for auxiliary measurement light in the axial direction Z is restricted as in the second embodiment.

### [Third embodiment]

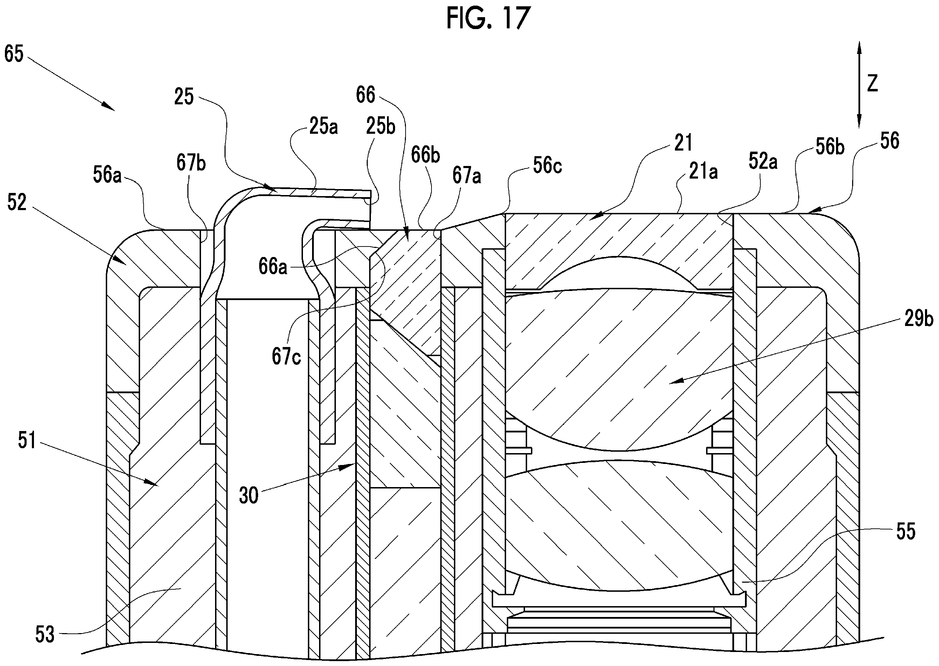

In the second embodiment, the lens 23 for auxiliary measurement light and the air/water supply nozzle 25 are caused to be in contact with each other so that the position of the lens 23 for auxiliary measurement light is restricted. However, in a third embodiment, a lens for auxiliary measurement light and a distal end cap are caused to be in contact with each other so that the position of the lens for auxiliary measurement light is restricted. In a distal end part 65 shown in Fig. 17, a lens 66 for auxiliary measurement light includes a notched portion 66a like the lens 61 for auxiliary measurement light of the second embodiment. Since components other than the lens 66 for auxiliary measurement light and through-holes 67a and 67b of a distal end cap 52 are the same as those of the distal end part 12d and the auxiliary measurement light-emitting unit 30 of the first embodiment, these components will be denoted by the same reference numerals and the description thereof will be omitted.

The notched portion 66a is formed at a position, which avoids the optical path of auxiliary measurement light emitted from the lens 66 for auxiliary measurement light, like the notch 61a of the lens 61 for auxiliary measurement light of the second embodiment. Accordingly, the outer shape of a spot SP, which is formed on a subject in a case where the subject is irradiated with the auxiliary measurement light, is not lost.

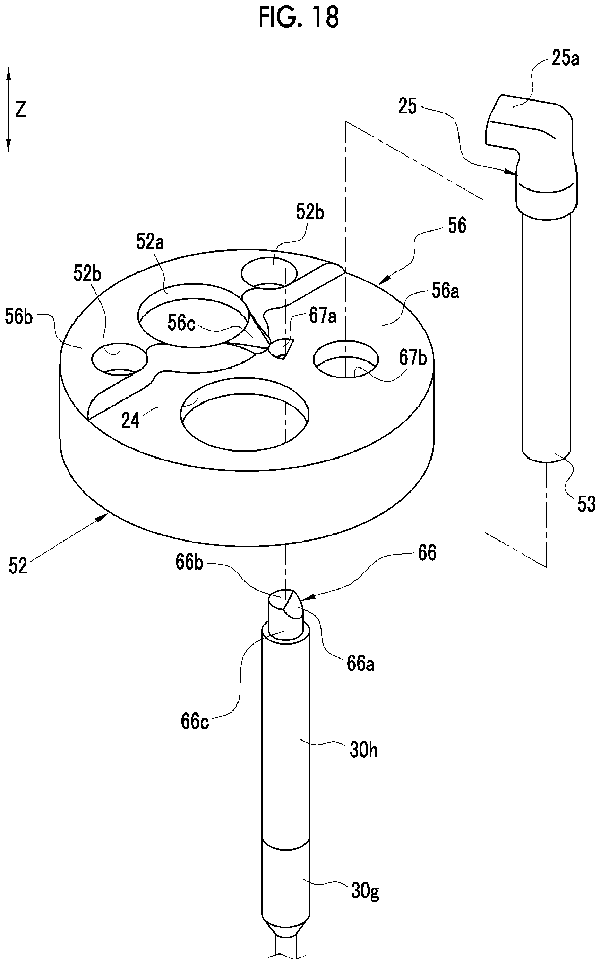

As shown in Fig. 18, the notched portion 66a is an inclined surface that is inclined toward an outer peripheral surface 66c from a distal end surface 66b of the lens 66 for auxiliary measurement light. Through-holes 67a and 67b are formed in the distal end cap 52, and allow the lens 66 for auxiliary measurement light and the air/water supply nozzle 25 to be exposed. The lens 66 for auxiliary measurement light includes the notched portion 66a that is provided at a position facing the proximal end side of the distal end cap 52. The through-hole 67a has a shape in which a part of a circular shape is notched to correspond to the shape of the distal end surface 66b. The notched portion 66a abuts on the proximal end side of the distal end cap 52, and the distal end surface 66b is exposed from the through-hole 67a. Further, an inclined surface 67c (see Fig. 17), which has an inclination corresponding to the notched portion 66a, is formed at the through-hole 67a. Accordingly, the position of the lens 66 for auxiliary measurement light in the axial direction Z is restricted.

Since the position of the lens 66 for auxiliary measurement light is restricted by the distal end cap 52 as described above, the lens 66 for auxiliary measurement light can be accurately positioned in the axial direction Z. Further, since the position of the lens 66 for auxiliary measurement light is restricted by the distal end cap 52, the separation of the lens 66 for auxiliary measurement light in the axial direction Z can be prevented. Furthermore, since the distal end side of the lens 66 for auxiliary measurement light does not need to be fixed to the distal end part body 51 by adhesion or the like in the assembly step, the number of steps of assembling the distal end part 65 can also be reduced.

Further, since the through-hole 67a is formed in a shape in which a part of a circular shape is notched as described above, the jetting port 25b of the air/water supply nozzle 25 can be disposed close to the distal end surface 66b of the lens 66 for auxiliary measurement light. Accordingly, the dirt of the lens 23 for auxiliary measurement light can be more efficiently removed.

Furthermore, an inclined surface, which is inclined toward the outer peripheral surface 66c from the distal end surface 66b, is formed on the lens 66 for auxiliary measurement light as the notched portion 66a in the second embodiment, but the present invention is not limited thereto. A notched portion, which is in contact with the distal end cap 52 in a case where the lens 66 for auxiliary measurement light is assembled in the distal end part 65, may be formed or, for example, a notched portion having a level difference, which is recessed from a distal end surface 66b and is parallel to the distal end surface 66b, may be formed as in the lens 63 for auxiliary measurement light shown in Fig. 16 of the second embodiment. Accordingly, the position of the lens 66 for auxiliary measurement light in the axial direction Z is restricted as in the third embodiment.

### [Modification examples]

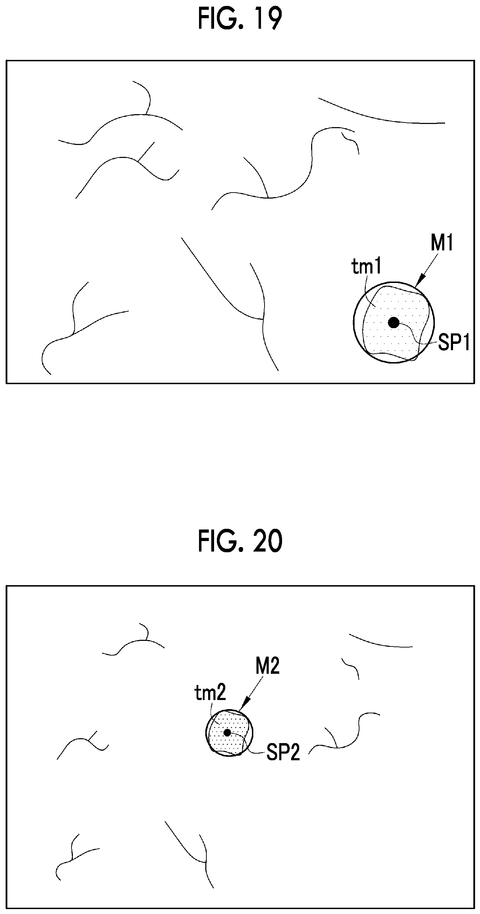

Various modification examples of each embodiment will be described below. The same components as those of each embodiment will be denoted by the same reference numerals and the description thereof will be omitted. In each embodiment, it has been described that a measurement marker to be used for the measurement of the size and the like of an object to be observed is displayed in a picked-up image. Specifically, the display controller 40 causes the monitor 18 to display a measurement image in which a measurement marker is displayed in a subject image on the basis of the position of the spot SP that is an irradiation region. More specifically, the display controller 40 causes the monitor 18 to display the measurement image in which the first measurement marker is superimposed to center on the spot SP. For example, a circular measurement marker is used as the first measurement marker. In this case, as shown in Fig. 19, a marker M1, which shows an actual size of 5 mm (a horizontal direction and a vertical direction of the subject image), is displayed at the center of a spot SP1 formed on a tumor tm1 of a subject in a case where an observation distance is close to the near end PN. In a case where the measurement marker is displayed on the monitor 18, an observation distance may also be displayed on the monitor 18 together.

Further, as shown in Fig. 20, a marker M2, which shows an actual size of 5 mm (the horizontal direction and the vertical direction of the subject image), is displayed at the center of a spot SP2 formed on a tumor tm2 of a subject in a case where an observation distance is close to the intermediate vicinity PM. Since the marker display position of the marker M2 is positioned at the central portion of the subject image that is less likely to be affected by distortion caused by the objective lens 21, the marker M2 has a circular shape without being affected by the distortion or the like.

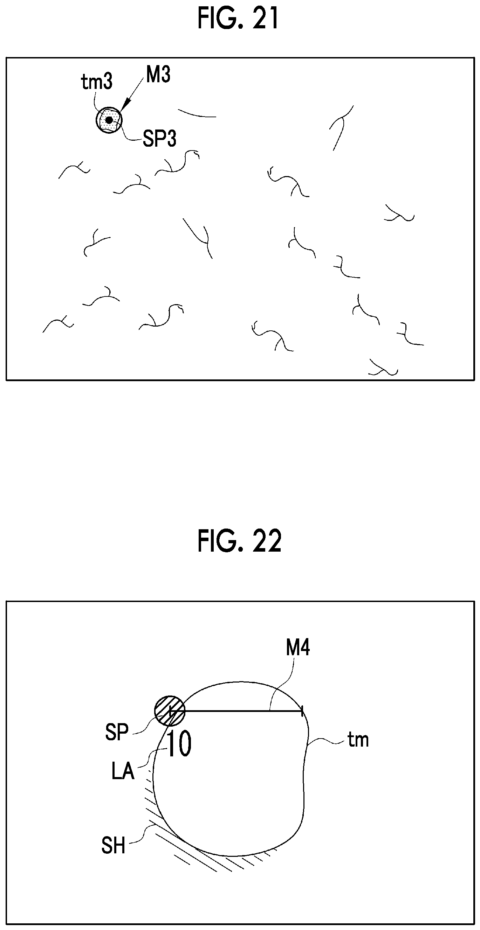

Furthermore, as shown in Fig. 21, a marker M3, which shows an actual size of 5 mm (the horizontal direction and the vertical direction of the subject image), is displayed at the center of a spot SP3 formed on a tumor tm3 of a subject. As shown in Figs. 19 to 21 having been described above, the size of the first measurement marker corresponding to the same actual size of 5 mm is reduced with an increase in an observation distance. Moreover, the shape of the first measurement marker also varies depending on the marker display position due to an influence of the distortion caused by the objective lens 21.

In Figs. 19 to 21, the center of the spot SP and the center of the marker are displayed to coincide with each other. However, the first measurement marker may be displayed at a position away from the spot SP in a case where there is no problem in measurement accuracy. Even in this case, it is preferable that the first measurement marker is displayed near the spot.

Further, the first measurement marker corresponding to the actual size of the subject, which is 5 mm, is displayed in Figs. 19 to 21, but the actual size of the subject may be set to any value (for example, 2 mm, 3 mm, 10 mm, or the like) according to an object to be observed or the purpose of observation.

A positional relationship between the measurement marker and the spot SP is not limited to a relationship in which the spot SP is positioned at any one of the "centroid", "center", or "coordinates regarded as the center" of the measurement marker as shown in Figs. 19 to 21, and the shape of the measurement marker is not limited to a circular shape. For example, as shown in Figs. 22 to 25, a measurement marker setting section 61 may set a measurement marker that corresponds to the position of the spot SP and includes gradations of which an end portion serves as a base point. The end portion is a portion closer to the outer portion than a central portion, or a starting point, an end point, or the like in the shape of the measurement marker.

Fig. 22 shows a measurement image in which a marker M4 set by the measurement marker setting section 61 is superimposed on a subject image so that the position of the spot SP and the base point of the gradations of the marker M4 overlap with each other. Since a tumor tm has a three-dimensional shape in Figs. 22 to 24 and Figs. 25A, 25B, and 25C, the subject image includes the tumor tm and the spot SP and includes a shadow SH in some cases. It is preferable that the marker M4 is superimposed to be displayed at the position of the spot SP for more accurate measurement. Accordingly, it is preferable that the marker M4 is displayed close to the spot SP as much as possible even in a case where the marker M4 is displayed at a position away from the spot SP. The marker M4 is a straight line segment, and includes gradations, which are line segments perpendicular to the straight line segment, at the starting point and the end point of the line segment. In a case where the marker M4 is a line segment or the like and has a starting point and an end point, the starting point and/or the end point themselves may be used as gradations. In this case, for example, gradations having the shape of a line segment perpendicular to the straight line segment may not be provided.

Further, the marker M4 may include a number "10" near the base point of the gradations. This is a gradation label LA of the marker M4, and is given so that the line segment of the marker M4 can be easily recognized as an actual size of 10 mm. Hereinafter, numbers included in measurement markers have the same meaning. The numerical value of the gradation label LA can be changed by setting, and a marker M4 of which a gradation label LA itself is not displayed may be provided.

Various types of measurement markers are used depending on settings. For example, a measurement marker having the shape of a straight line segment or a shape in which straight line segments are combined with each other, a measurement marker having a circular shape or a shape in which circles are combined with each other, or a measurement marker having a shape in which a circle and a straight line segment are combined with each other, and the like are used.

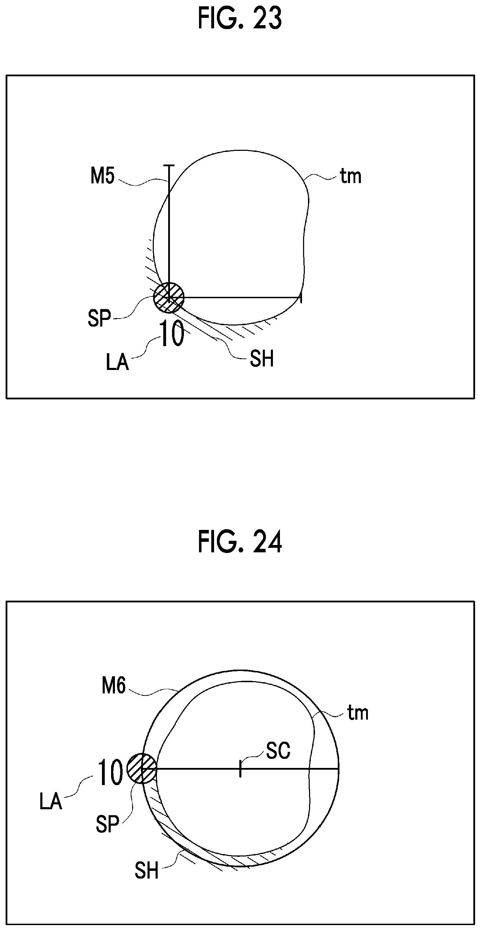

For example, a measurement image shown in Fig. 23 includes a marker M5 having a shape in which straight line segments are combined with each other. The marker M5 has a shape in which straight line segments are combined in an L shape, the line segments extend upward along the plane of paper and in a direction along the plane of paper from the corner of the L shape as a base point, and the marker M5 includes a gradation at each of end points of the line segments with the base point as a starting point. Further, the marker M5 includes a number "10", which is a gradation label LA, near the base point of the gradations like the marker M4.

For example, a measurement image shown in Fig. 24 includes a marker M6 having a shape in which a straight line segment and a circle are combined with each other. The marker M6 has a shape in which a circle and a line segment corresponding to the diameter of the circle are combined with each other, and the line segment extends to the right along the plane of paper from one of intersections between the line segment and the circle as a base point. With regard to the line segment or the circle, the respective intersections between the line segment and the circle are used as gradations. The marker M6 may include a gradations SC at a point where the line segment is divided in half or the center of the circle. Further, the marker M6 includes a number "10", which is a gradation label LA, near the base point of the gradations like the marker M4 or the marker M5.

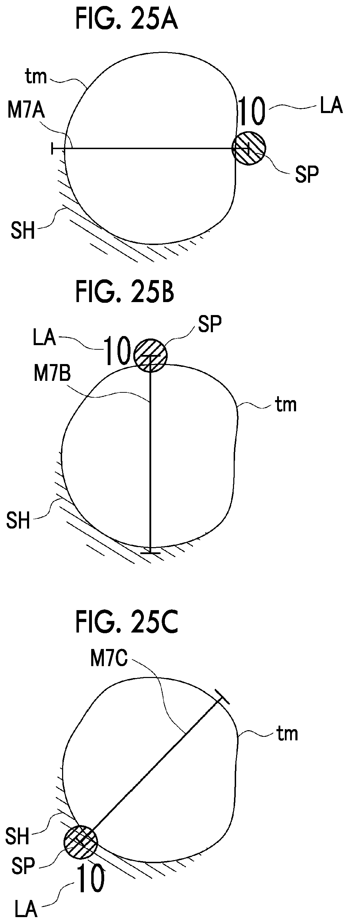

As shown in Figs. 25A, 25B, and 25C, in addition to these, the measurement marker may have various shapes like, for example, a marker M7A (Fig. 25A) of which a line segment extends from a base point to the left along the plane of paper and which includes a gradation label LA, a marker M7B (Fig. 25B) of which a line segment extends downward along the plane of paper from a base point and which includes a gradation label LA, a marker M7C (Fig. 25C) of which a line segment extends in a direction oblique to the upper right side along the plane of paper from a base point and which includes a gradation label LA, or the like.

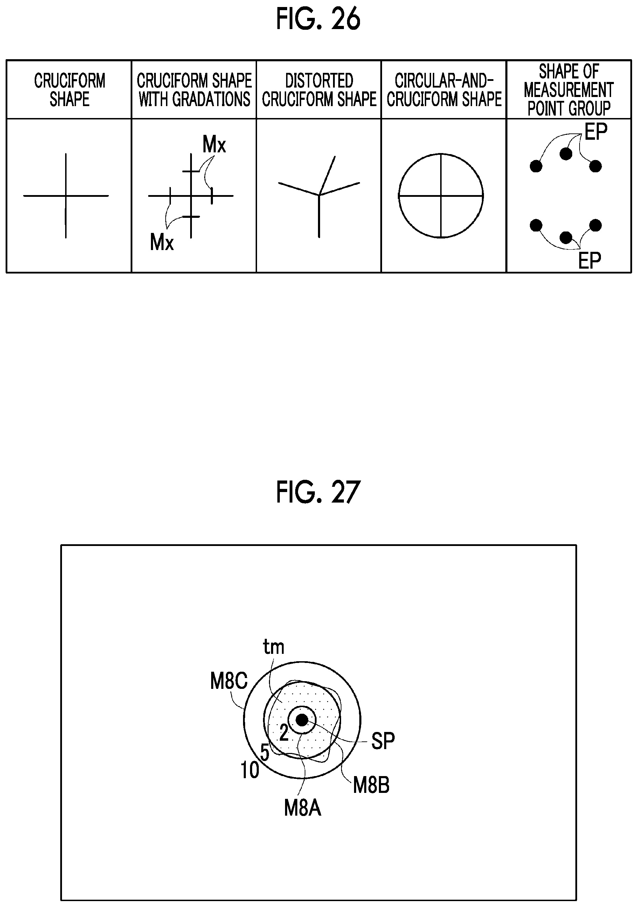

Further, the first measurement marker may have a cruciform shape where a vertical line and a horizontal line intersect with each other as shown in Fig. 26 in addition to these shapes. Furthermore, the first measurement marker may have a cruciform shape with gradations where gradations Mx are given to at least one of a vertical line or a horizontal line of a cruciform shape. Further, the first measurement marker may have a distorted cruciform shape of which at least one of a vertical line or a horizontal line is inclined. Furthermore, the first measurement marker may have a circular-and-cruciform shape where a cruciform shape and a circle are combined with each other. In addition, the first measurement marker may have the shape of a measurement point group where a plurality of measurement points EP corresponding to an actual size from a spot are combined with each other. Further, one first measurement marker may be displayed or a plurality of first measurement markers may be displayed, and the color of the first measurement marker may be changed according to an actual size.

As shown in Fig. 27, three concentric circular markers M8A, M8B, and M8C having different sizes (diameters as the sizes are 2 mm, 5 mm, and 10 mm, respectively) may be displayed in the subject image as the first measurement marker to center on a spot SP formed on a tumor tm. Since the three concentric circular markers are displayed as a plurality of markers, time and effort required to switch a marker can be saved and measurement can be performed even in a case where a subject has a non-linear shape. In a case where a plurality of concentric circular markers are to be displayed to center on a spot, a size and a color are not designated for each marker and combinations of a plurality of conditions may be prepared in advance and one can be selected from these combinations.

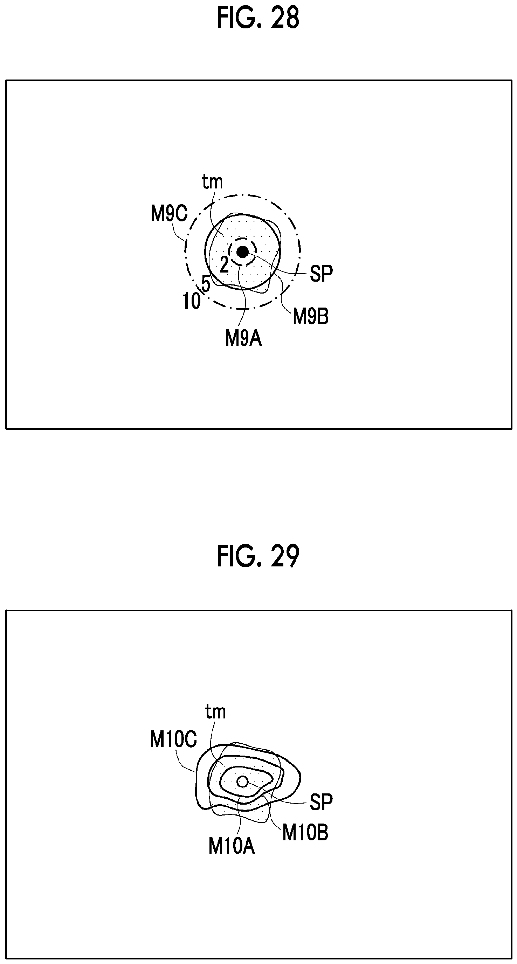

In Fig. 27, all the three concentric circular markers are displayed with the same color (black). However, in a case where a plurality of concentric circular markers are to be displayed, a plurality of color concentric circular markers of which colors are different from each other may be used. As shown in Fig. 28, a marker M9A is displayed by a dotted line representing a red color, a marker M9B is displayed by a solid line representing a blue color, and a marker M9C is displayed by a one-dot chain line representing a white color. Since identifiability can be improved in a case where the colors of the markers are different from each other in this way, measurement can be easily performed.

Further, as shown in Fig. 29, a plurality of distorted concentric circular markers, which are distorted from the respective concentric circles, may be used as the first measurement marker in addition to the plurality of concentric circular markers. In this case, distorted concentric circular markers M10A, M10B, and M10C are displayed in the subject image to center on a spot SP formed on a tumor tm.

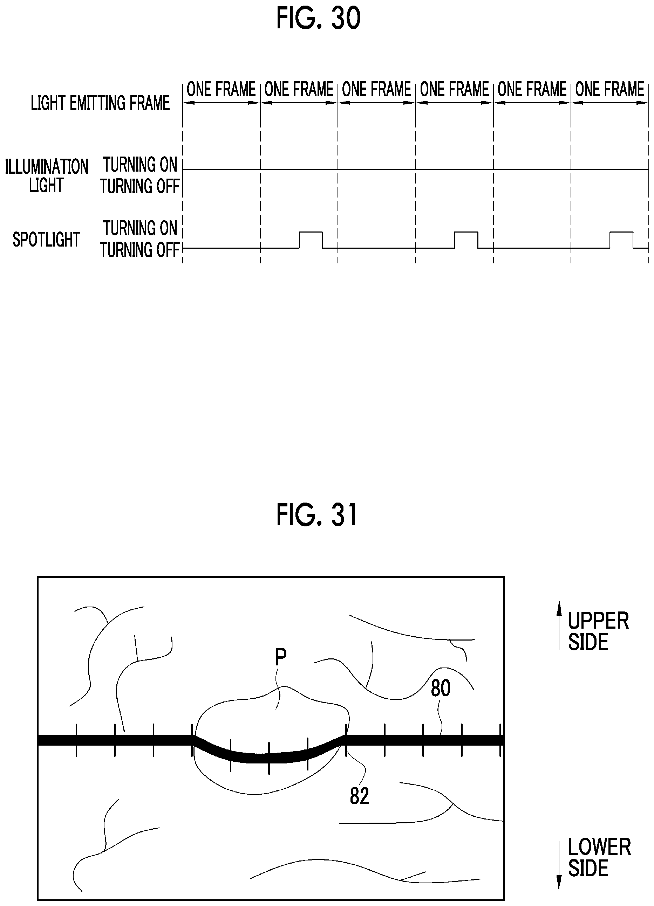

The subject is constantly irradiated with the illumination light and spotlight (measurement light) in the length measurement mode. However, as shown in Fig. 30, the illumination light may be constantly turned on so that the subject is irradiated with the illumination light and the spotlight may be repeatedly turned on and turned off (dimmed) every frame (or every few frames) so that the subject is intermittently irradiated with the spotlight. In this case, in a frame where the spotlight is turned on, the position of the spotlight is detected and the display of a measurement marker is set. Further, it is preferable that the measurement marker of which the display is set is superimposed and displayed on an image obtained in a frame where the subject is irradiated with only the illumination light.

Light, which forms a spot in a case where a subject is irradiated with the light, is used as the measurement light, but other light may be used. For example, line-like measurement light, which is formed on the subject as an intersection line 80 as shown in Fig. 31 in a case where the subject is irradiated with the light, may be used. In a case where the subject is irradiated with the line-like measurement light, the intersection line 80 as a line-like irradiation region is formed on the subject. In this case, a second measurement marker that consists of the intersection line 80 and gradations 82 formed on the intersection line 80 and serving as an index of the size of the subject (for example, a polyp P) is generated as a measurement marker.

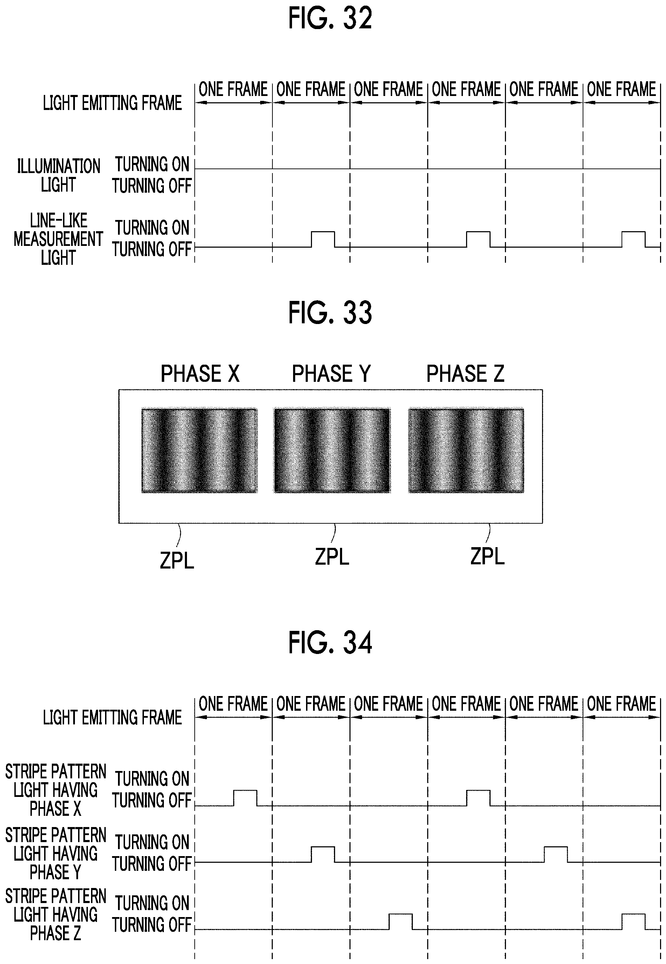

In a case where the line-like measurement light is used as the measurement light, in the length measurement mode, the subject may be constantly irradiated with the illumination light and the line-like measurement light or, as shown in Fig. 32, the subject may be constantly irradiated with the illumination light and the line-like measurement light may be repeatedly turned on and turned off (dimmed) every frame (or every few frames) so that the subject is intermittently irradiated with the line-like measurement light. In this case, in a frame where the line-like measurement light is turned on, the position of the line-like measurement light is detected and the display of a measurement marker is set. Further, it is preferable that the measurement marker of which the display is set is superimposed and displayed on an image obtained in a frame where the subject is irradiated with only the illumination light.

Stripe pattern light ZPL, which is formed as light having a stripe pattern on a subject as shown in Fig. 33 in a case where the subject is irradiated with the light, may be used as the measurement light (for example, see JP2016-198304A). The stripe pattern light ZPL is obtained in a case where a variable-transmittance liquid crystal shutter (not shown) is irradiated with specific laser light, and is formed of two different vertical stripe patterns in which a region (transmissive region) through which specific laser light is transmitted by the liquid crystal shutter and (non-transmissive region) through which the specific laser light is not transmitted are periodically repeated in the horizontal direction. In a case where the stripe pattern light is used as the measurement light, the period of the stripe pattern light is changed depending on a distance from the subject. Accordingly, the subject is irradiated with the stripe pattern light plural times while the period or phase of the stripe pattern light is shifted by the liquid crystal shutter, and the three-dimensional shape of the subject is measured on the basis of a plurality of images obtained from the shift of the period or phase.

For example, a subject is alternately irradiated with a stripe pattern light having a phase X, a stripe pattern light having a phase Y, and a stripe pattern light having a phase Z. The phases of the vertical stripe patterns of the stripe pattern lights having the phases X, Y, and Z are shifted from each other by 120° (2π/3). In this case, the three-dimensional shape of the subject is measured using three types of images obtained on the basis of the respective stripe pattern lights. For example, it is preferable that the subject is irradiated with the stripe pattern light having the phase X, the stripe pattern light having the phase Y, and the stripe pattern light having the phase Z while the stripe pattern light having the phase X, the stripe pattern light having the phase Y, and the stripe pattern light having the phase Z are switched every frame (or every few frames) as shown in Fig. 34. It is preferable that the subject is constantly irradiated with the illumination light.

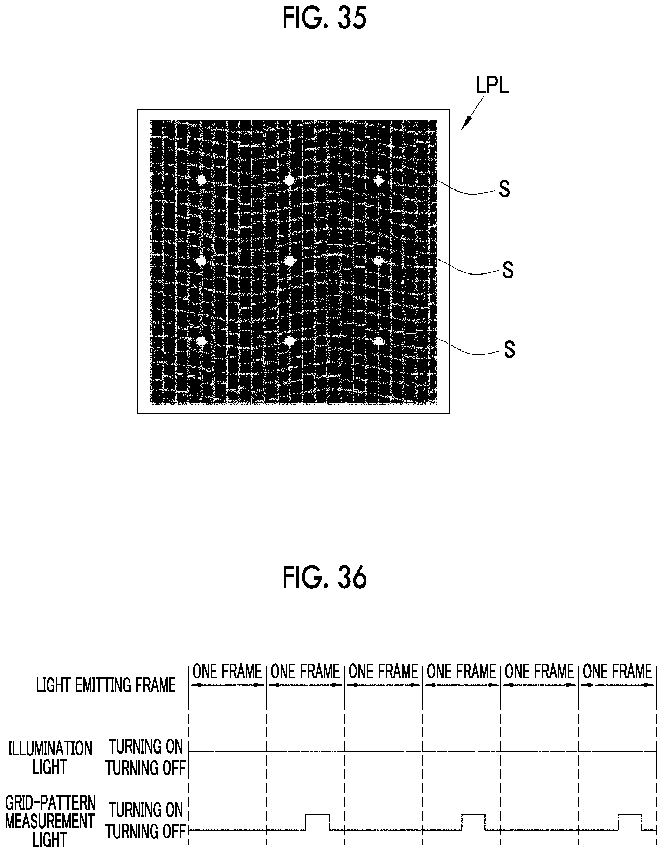

Grid-pattern measurement light LPL, which is formed as a grid pattern as shown in Fig. 35 in a case where a subject is irradiated with the light, may be used as the measurement light (for example, see JP2017-217215A). In this case, since the three-dimensional shape of the subject is measured according to the state of deformation of the grid pattern in a case where the subject is irradiated with the grid-pattern measurement light LPL, it is required to accurately detect the grid pattern. For this reason, the shape of the grid-pattern measurement light LPL is not a perfect grid shape and is set to a wave shape or the like, that is, is slightly deformed from a grid shape to improve the detection accuracy of the grid pattern. Further, the grid pattern is provided with S codes indicating that the end points of the left and right horizontal lines are continuous. In a case where the grid pattern is detected, not only the pattern but also the S codes are detected to improve the detection accuracy of the pattern. The grid pattern may be a pattern in which a plurality of spots are arranged in a grid shape in the vertical and horizontal directions in addition to a pattern in which vertical lines and horizontal lines are regularly arranged.

In a case where the grid-pattern measurement light LPL is used as the measurement light, in the length measurement mode, the subject may be constantly irradiated with the illumination light and the grid-pattern measurement light LPL or, as shown in Fig. 36, the subject may be constantly irradiated with the illumination light and the grid-pattern measurement light LPL may be repeatedly turned on and turned off (dimmed) every frame (or every few frames) so that the subject is intermittently irradiated with the grid-pattern measurement light LPL. In this case, the three-dimensional shape is measured on the basis of the grid-pattern measurement light LPL in a frame where the grid-pattern measurement light LPL is turned on. Further, it is preferable that the measurement result of the three-dimensional shape is superimposed and displayed on an image obtained in a frame where the subject is irradiated with only the illumination light.

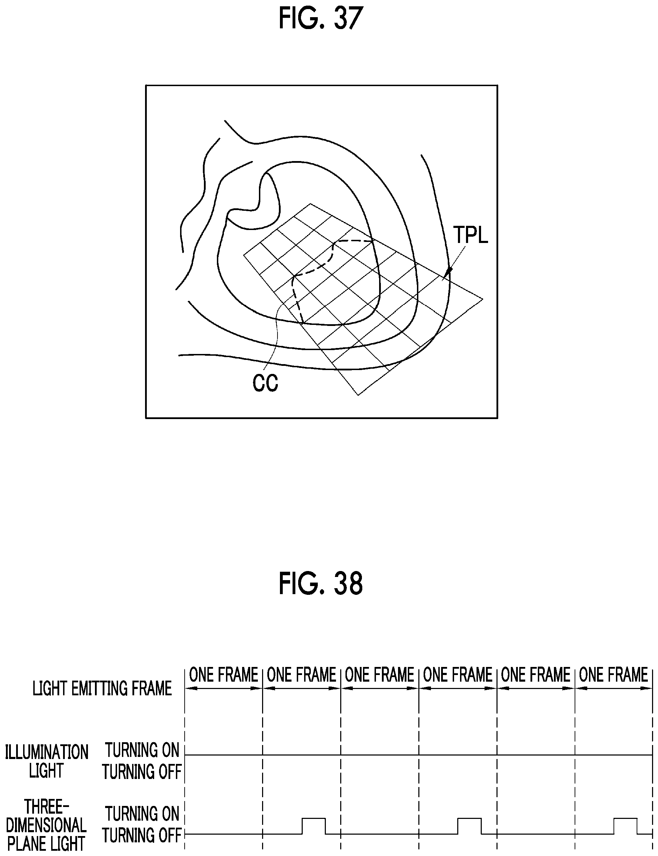

Three-dimensional plane light TPL, which is represented in a subject image by mesh lines as shown in Fig. 37, may be used as the measurement light (for example, see JP2017-508529A). In this case, a user moves the distal end part 12d so that the three-dimensional plane light TPL is aimed at an object to be measured. Then, in a case where the three-dimensional plane light TPL intersects with the object to be measured, a distance from an intersection curve CC between the three-dimensional plane light TPL and the subject is calculated by processing based on a manual operation of the user interface or the like, or automatic processing.

In a case where the three-dimensional plane light TPL is used as the measurement light, in the length measurement mode, the subject may be constantly irradiated with the illumination light and the three-dimensional plane light TPL or, as shown in Fig. 38, the subject may be constantly irradiated with the illumination light and the three-dimensional plane light TPL may be repeatedly turned on and turned off (dimmed) every frame (or every few frames) so that the subject is intermittently irradiated with the three-dimensional plane light TPL.

In each of the embodiments, the hardware structures of processing units, which perform various types of processing, such as the signal processing unit 39, the display controller 40, and the system controller 41, are various processors to be described below. Various processors include: a central processing unit (CPU) that is a general-purpose processor functioning as various processing units by executing software (programs); a graphical processing unit (GPU); a programmable logic device (PLD) that is a processor of which the circuit configuration can be changed after manufacture, such as a field programmable gate array (FPGA); a dedicated electrical circuit that is a processor having circuit configuration designed exclusively to perform various types of processing; and the like.

One processing unit may be formed of one of these various processors, or may be formed of a combination of two or more same type or different types of processors (for example, a plurality of FPGAs, a combination of a CPU and an FPGA, a combination of a CPU and a GPU, or the like). Further, a plurality of processing units may be formed of one processor. As an example where a plurality of processing units are formed of one processor, first, there is an aspect where one processor is formed of a combination of one or more CPUs and software as typified by a computer, such as a client or a server, and this processor functions as a plurality of processing units. Second, there is an aspect where a processor fulfilling the functions of the entire system, which includes a plurality of processing units, by one integrated circuit (IC) chip as typified by System On Chip (SoC) or the like is used. In this way, various processing units are formed using one or more of the above-mentioned various processors as hardware structures.

In addition, the hardware structures of these various processors are more specifically electrical circuitry where circuit elements, such as semiconductor elements, are combined.

### Explanation of References

10: endoscope system
12: endoscope
12a: insertion part
12b: operation part
12c: bendable part
12d: distal end part
12e: angle knob
12f: treatment tool inlet port
12g: suction button
12h: air/water supply button
13a: mode changeover switch
13b: freeze switch
14: light source device
16: processor device
18: monitor
19: user interface
21: objective lens
21a: distal end surface
22: illumination lens
22a: distal end surface
23: lens for auxiliary measurement light
23a: distal end surface
23b: proximal end surface
24: treatment tool outlet
25: air/water supply nozzle
25a: jetting cylinder portion
25b: jetting port
26: light source unit
27: light source controller
28: light guide
29a: illumination optical system
29b: image pickup optical system
30: auxiliary measurement light-emitting unit
30a: light source
30b: lens
30b: gradient index (GRIN) lens
30c: prism
30d: optical fiber
30e: fiber sheath
30f: ferrule
30g: reinforcing material
30h: housing
32: image pickup element
33: image pickup controller
34: CDS/AGC circuit
35: A/D
36: communication interface (I/F)
38: communication interface (I/F)
39: signal processing unit
40: display controller
41: system controller
42: static image storage unit
43: static image storage controller
45: solid line
46: dotted line
51: distal end part body
52: distal end cap
52a: through-hole
52b: through-hole
52c: through-hole
52d: through-hole
53: connection pipe
54: treatment tool-insertion tube
55: lens barrel
56: distal end surface
56a: flat surface
56b: flat surface
56c: guide surface
57: air/liquid supply tube
60: distal end part
61: lens for auxiliary measurement light
61a: notched portion
61b: distal end surface
61c: outer peripheral surface
62a: through-hole
62b: through-hole
63: lens for auxiliary measurement light
63a: notched portion
63b: distal end surface
65: distal end part
66: lens for auxiliary measurement light
66a: notched portion
66b: distal end surface
66c: outer peripheral surface
67a: through-hole
67b: through-hole
67c: inclined surface
80: intersection line
82: gradation
120: distal end part
121: objective lens
125: air/water supply nozzle
CA: central axis
CA2: central axis
CL: center line
d1: outer diameter
D1: first direction
d12: outer diameter
d2: outer diameter
D2: second direction
DOE: light source
F1: flow velocity
F2: flow velocity
G1: first minimum distance
G2: second minimum distance
H: subject
LD: laser light source
LI: optical axis
LM: optical axis
LM2: optical axis
LPL: grid-pattern measurement light
M1, M2, M3, M4, M5, M6, M7A, M7B, M7C, M8A, M8B, M8C, M9A, M9B, M9C, M10A, M10B, M10C: marker
Mx: gradation
PF: far end
PM: intermediate vicinity
PN: near end
QF: arrow
QM: arrow
QN: arrow
R1: range
SC: gradation
SH: shadow
SP, SP1, SP2, SP3: spot
tm, tm1, tm2, tm3: tumor
TPL: three-dimensional plane light
W1, W11: opening width
Z: axial direction
ZPL: stripe pattern light

## Claims

1. An endoscope comprising:
an insertion part that is to be inserted into an object to be examined;
a distal end surface that is provided at a distal end of the insertion part;
an observation window that is disposed on the distal end surface;
a fluid jet nozzle that is disposed on the distal end surface and jets fluid toward the observation window; and
an auxiliary light-emitting window that is disposed on the distal end surface and emits auxiliary measurement light,
wherein the auxiliary light-emitting window is disposed between the observation window and the fluid jet nozzle in a fluid jet range of the fluid jet nozzle.

2. The endoscope according to claim 1,
wherein the fluid jet nozzle jets liquid or gas as the fluid, and
in a case where the liquid or the gas is jetted toward the observation window from the fluid jet nozzle, a flow velocity of the liquid at a position where the liquid reaches the observation window is 2 m/s or more and a flow velocity of the gas at a position where the gas reaches the observation window is 40 m/s or more.

3. The endoscope according to claim 1 or 2,
wherein an optical member for an auxiliary light-emitting window, which forms the auxiliary light-emitting window, includes a notched portion that is provided at a position facing the fluid jet nozzle.

4. The endoscope according to claim 3,
wherein the notched portion is disposed at a position that avoids an optical path of the auxiliary measurement light in a case where the auxiliary measurement light is emitted from the auxiliary light-emitting window.

5. The endoscope according to claim 3 or 4,
wherein the fluid jet nozzle is assembled in the insertion part in a state where the fluid jet nozzle is in contact with the notched portion.

6. The endoscope according to claim 5,
wherein the notched portion is in contact with the fluid jet nozzle, so that a position of the auxiliary light-emitting window in an axial direction of the insertion part is restricted.

7. The endoscope according to claim 1 or 2, further comprising:
a distal end part body that holds an optical member for an auxiliary light-emitting window forming the auxiliary light-emitting window, an image pickup optical system including the observation window, and the fluid jet nozzle; and
a distal end cap that covers a distal end side of the distal end part body,
wherein the optical member for an auxiliary light-emitting window includes a notched portion that is provided at a position facing the distal end cap.

8. The endoscope according to claim 7,
wherein the notched portion is in contact with the distal end cap, so that a position of the auxiliary light-emitting window in an axial direction of the insertion part is restricted.

9. The endoscope according to any one of claims 3 to 8,
wherein the optical member for an auxiliary light-emitting window is formed in a columnar shape, and
the notched portion is an inclined surface that is inclined toward an outer peripheral surface from a distal end of the optical member for auxiliary measurement light.

10. The endoscope according to any one of claims 1 to 9,
wherein an outer diameter of the auxiliary light-emitting window is 0.5 mm or more and 1.6 mm or less,
a first minimum distance, which is a minimum distance between an outer peripheral edge of the observation window and an outer peripheral edge of the auxiliary light-emitting window, is 0 mm or more and 1.5 mm or less, and
a second minimum distance, which is a minimum distance between the outer peripheral edge of the auxiliary light-emitting window and a distal end of the fluid jet nozzle, is 0 mm or more and 0.5 mm or less.

11. The endoscope according to any one of claims 1 to 10,
wherein a mounting position for the fluid jet nozzle on the distal end surface and a distal end surface of the auxiliary light-emitting window are disposed at a same position in an axial direction of the insertion part, and
a distal end surface of the observation window is positioned on a distal end side of the distal end surface of the auxiliary light-emitting window in the axial direction and includes a guide surface that is continuous between an outer peripheral edge of the auxiliary light-emitting window and an outer peripheral edge of the observation window.

12. The endoscope according to claim 11,
wherein an opening width of the fluid jet nozzle is smaller than an outer diameter of the observation window, and
the guide surface is positioned in the fluid jet range of the fluid jet nozzle.

13. The endoscope according to any one of claims 1 to 12,
wherein an outer diameter of the auxiliary light-emitting window is smaller than an outer diameter of the observation window.
